(19) Europäisches Patentamt
European Patent Office
Office européen des brevets

(11) **EP 4 296 351 A1**

(12) **EUROPEAN PATENT APPLICATION**

(43) Date of publication:
**27.12.2023  Bulletin 2023/52**

(21) Application number: **23179466.0**

(22) Date of filing: **15.06.2023**

(51) International Patent Classification (IPC):
**C12M 3/00** $^{(2006.01)}$     **C12M 1/34** $^{(2006.01)}$
**G01N 27/22** $^{(2006.01)}$

(52) Cooperative Patent Classification (CPC):
**C12M 41/36; C12M 23/48; C12M 41/46**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.06.2022  DK PA202200590**

(71) Applicant: **Dansk Fundamental Metrologi A/S
2970 Hørsholm (DK)**

(72) Inventors:
• **Aslan, Hüsnü
  2970 Hørsholm (DK)**
• **Nielsen, Ole Stender
  2970 Hørsholm (DK)**
• **Thirstrup, Carsten
  2970 Hørsholm (DK)**

(74) Representative: **Plougmann Vingtoft a/s
Strandvejen 70
2900 Hellerup (DK)**

(54)   **NON-CONTACT IMPEDANCE ANALYZER FOR REAL-TIME DETECTION OF MICROBIAL GROWTH**

(57)   A device and method for real-time quantitative detection of growth of microorganisms in one or more containers through one or more electrically insulated walls of each container is provided with at least one set of electrodes physically separated from each other and being adapted to establish one or more contact interfaces with insulated walls of each container. The device comprises a docking station, which is adapted to attach and detach each container, ensuring that the electrodes are positioned at the contact interfaces with predefined positions. The device further comprises at least one set of one or more electrical connectors adapted for electrical connections to the sets of electrodes. Connecting the device to an impedance analyser enables growth of microorganisms to be detected in real time with no physical contact between the samples of microorganisms and the electrodes.

Figure 1

**EP 4 296 351 A1**

## Description

### Field of the invention

The present invention is within the field of real-time detection of growth of microorganisms such as bacteria or biofilms in containers with insulated walls of commercial labware such as laboratory flasks, microwell plates and laboratory vials, dishes etc. More specifically, embodiments of the present invention are a docking system adapted to dock containers with electrically insulated walls and comprising microorganisms reproducibly into a docking station of a docking system. Positioning electrodes of the docking system at preselected positions on the insulated walls of the containers enables reproducible real-time analyses of growth of microorganisms by an impedance analyser connected to the device.

### Background of the invention

When growing bacteria in biotechnology applications, microorganisms and their by-products need to be sampled and analysed [C.E. Turick et al. Appl. Microbiol. Biotechnol. 103, 8327-8338 (2019)]. Commonly used methods include biochemical phenotype methods, molecular hybridization methods and amplification methods [M. Simic et al. IEEE Sensors J. 20, 12791-12797 (2020)], but they are time consuming, labour intensive and costly.

Bacteria have a wide variety of shapes, dimensions and compositions [R. Gnaim et al. BioTechniques 69, 27-36 (2020)]. Various techniques have been developed for faster and less labour-intensive determination of bacterial concentration, including fluorescence cytometry (WO 95/31481) and image-analysis (US 8 780 181). Other methods are cell counting, Coulter counters, pH monitoring, magnetic, electrochemical and bioluminescence [R. Narang et al. Sci. Rep. 8, 15807 (2018)]. These techniques require extensive image or signal processing as well as large and expensive instrumentation, which is laborious to work with and lack the potential for miniaturization. In addition, special effort is required to manufacture custom systems in which electrodes are exposed to growing bacteria.

Non-labelling detection methods are simpler to work with and include quartz crystal microbalance and surface plasmon resonance techniques. However, those methods also require that the bacterial growth platforms are adapted to these instruments.

During bacterial growth, the structural and functional changes that occur to a cell during growth can be detected by electrochemical analysers, which enable the monitoring of the bioprocesses in real-time. Applying electrical signals to living cells causes reactions with the components of the cells and frequency dependent polarization, which can be related to the polar nature of lipid membranes, proton gradients on the outer surface of viable cells, and membrane-associated electron transfer reactions linked to metabolic activity. Simplified, this behaviour of the cells can be described in terms of capacitance, electrical permittivity and conductivity. The capacitance to resistance ratio changes during cellular growth and reflects factors related to biomass density, cell viability, membrane integrity and the overall metabolic state of microbes. Dead cells or non-biomass solids that do not pose an intact plasma membrane do not polarize and therefore do not have a significant capacitance relative to the cell suspension [J. Song et al. ACS Sens. 5, 1795-1803 (2020)].

The variation in capacitance and resistance can readily be measured by an analyser using electrochemical-impedance-spectroscopy (EIS). EIS is a fast, sensitive, and label-free technique that can characterize (bio)physical processes at the substrate-biomaterial interface. For standard impedance spectra, at low frequencies (up to several tens of kHz), the effects of a double layer (metal-medium interface effects) are dominant. At medium frequencies (several tens of kHz to several MHz), the impedance depends on the medium and cell/particle. There is a first plateau, which depends on cell size, and a second plateau, which depends on cell cytoplasm properties. At frequencies above several MHz, the parasitic effects due to the substrate and electrode connections are dominant [A. L. A. de Araujo et al. Biosensors 9, 108, 1-4 (2019)]. However, for analysers based on EIS, microbial growth on surfaces has been demonstrated to be sensitive to the choice of metal electrode material such as stainless steel, indium-tin-oxide, platinum and gold [J. Song et al. ACS Sens. 5, 1795-1803 (2020)].

An online monitoring of bacterial growth based on a multichannel capacitively coupled contactless conductivity detector and eight bacterial culture tubes, where the electrodes are not in direct contact with the measured solutions has been reported [Zhang et al., Anal. Chem. 2018, 90, 10, 6006-6011]. The detector for each tube is based on a couple of copper cylinders with internal diameter of 5.01, thickness of 0.4 mm and a length of 20 mm and with a spacing of 46.0 mm. GB 2 260 407 describes non-contacting, bridgeless methods which utilize concentric multi-electrode or multi-coil structures for capacitive and inductive measurement on samples of matter without contact together with the use of multiple-frequency excitations to separate out or reduce effects of ionic conductivity and/or multiple dielectric dispersions in samples. From any such dispersion or frequency region of interest, the methods yield physical or chemical properties of the sample, e.g. biomass in water. However, the method described in GB 2 260 407 is not suitable for detecting growth of microorganisms in off-the-shelf labware.

US 2006/0216203 describes a multi-well impedance measurement device for real-time detection of cellular activation with a plurality of wells and/or chambers and with a plurality of electrodes lying on the bottom surface of each plurality of wells/chambers. However, the electrodes are exposed to the samples as measured by the device. The system is based on contact impedance measuring electrodes, where the electrodes are in physical contact with the samples, and they cannot be attached to and detached from the wells comprising the cells growing at the bottom of the wells.

US 2009/0149334 describes a method of measuring properties of a liquid contained in individual wells inside a multi-well array, where capacitor electrodes are provided in the multi-well array. The method is based on measuring capacitance inside each individual well, where the electrodes are adapted to detect a capacitance value of each individual well without interference of neighbouring wells. The property can be a volume of the liquid inside the individual wells, a state of presence of the liquid inside the individual wells or further a change of volume of the liquid inside the individual wells. However, this invention is not applicable to wells with insulated walls in off-the-shelf labware.

EP 3 301 438 describes a sensor and method for detecting a state of a fluid within a microfluidic plate, comprising at least a first planar electrode and a second planar electrode arranged parallel to one another to form an electric capacitor and configured to generate an electric field within the fluid. The sensor is based on detecting the state of the fluid by means of a capacitance of the electric capacitor.

The state of the fluid comprises at least one element of the group consisting of presence of the fluid, absence of the fluid, predetermined mixture of the fluid with at least one component different from the fluid, presence of an enclosure of the fluid, absence of an enclosure of the fluid, fill level of the fluid, and flow rate of the fluid. The fluid may be a liquid, particularly a biological liquid. The sensor comprises planar electrodes, which do not contact the fluid and may be arranged parallel to the microfluidic plate.

However, EP 3 301 438 does not teach how to attach and detach the connection between the wells comprising the fluid and the planar electrodes and how the electrodes are positioned with respect to the microfluidic plate comprising the fluid, which is required for a reliable detection of growth of microorganisms. In addition, the invention is not applicable to off-the-shelf labware.

US 9400272 describes a non-invasive method of detecting microbial growth by monitoring changes in the dielectric constant of the specimen caused by metabolic processes. Changes in the dielectric constant of a biological sample in a growth medium or minute changes to a biological sample's dielectric constant are measured e.g. by varying the electric field capable of sensing a change in the dielectric constant between capacitive electrodes.

In one embodiment, the interdigitated electrodes are provided on a flexible circuit substrate such as mylar or Kevlar film, and the electrodes are wrapped around the vessel or bottle such that an electrode surface area is in physical proximity to the liquid sample inside, where the dielectric constant of the sample changes with microorganism growth and metabolite release. In another embodiment, plates include opposing pairs of conductive electrodes, wherein each pair of electrodes are placed above and below microwells. However, US 9400272 does not teach how to reproducibly attach and detach the vials, vessels, bottles or microwells with respect to the electrodes and how to minimize and/or reproduce the spacing between the electrodes and the surfaces of the vials, vessels, bottles of microwells by establishing contact interfaces. Since any variations in the electrode positions and spacing will cause a change in the capacitance, US 9400272 is not adapted to make quantitative measurements of microorganisms. In addition, the invention is not applicable to off-the-shelf labware.

US patent application 2019/0359970 describes an invention of a cuvette comprising a body of a nonconductive material, a sample cavity configured to receive a sample, a first wall configured to couple to a first electrode of a pulse generating system that comprises a capacitive element, and a second wall configured to couple to a second electrode of a pulse generating system. In some embodiments of the invention, the cuvette is a container configured to hold a sample which may be disposed between two electrodes, which are spaced apart on opposite sides of the container, and a sample holder may a spring-loaded mechanism that pushes electrodes of the sample holder against the two electrodes. However, in these embodiments, the cuvette comprises electrodes in physical contact with the sample to be measured, and the invention is not applicable to off-the-shelf labware.

## Summary of the invention

There is a need of rapid, real-time, high-throughput and non-contact detection of microbial growth, which can be used with off-the-shelf labware with insulated walls, including laboratory bottles, flasks, T-flasks, vials, dishes and plates. There is therefore a need of a device enabling the attachment and detachment of containers comprising microorganism as integrated with off-the-shelf labware to an analyser for real-time detection of microbial growth reproducibly. There is also a need of ensuring reproducibly positioning and with minimized spacing between the electrodes and the insulated walls of the containers and with no physical contact between the electrodes and the microorganisms. It has been found that this objective may be achieved by means of locating at least one of the electrodes at the bottom side of a container, thereby making it readily to attach and detach the electrodes to and from the container reproducibly with minimum spacing between the electrodes and the outer surface of the container. This enables reproducibly quantitative determination of growth of microorganisms.

These and other object needs have been fulfilled by the invention or embodiments thereof as defined in the claims and/or as described herein below.

It has been found that the invention or embodiments thereof have a number of additional advantages, which will be clear to the skilled person from the following description.

In an embodiment of the present invention of a docking system for electrochemical detection of growth of microorganisms in a container, a docking system comprises a docking station, a set of electrodes comprising at least two electrodes and a docking arrangement. The docking arrangement comprises at least one movable element and a set of electrical connectors adapted for connecting the respective electrodes to analyser, and the docking arrangement is adapted for temporarily holding a container in location relative to the set of electrodes, to provide the respective electrode to be in physical contact with an outer surface of said container at respective preselected locations, wherein the electrodes are physically separated from each other. The docking arrangement is connected to or is connectable to the docking station, and the docking station may comprise a docking site for a container, wherein the docking site may comprise a bottom structure comprising at least one electrode of the set of electrodes.

In another embodiment of the present invention of a docking system, one or more electrodes of the set of electrodes are stationary in the docking station at a docking site to ensure that the electrode comes into physical contact with the outer surface of a container when located at the docking site in the docking station, and the stationary electrode preferably forms part of a bottom structure of the docking site.

The docking arrangement in the docking system may be correlated to or engaged with at least one of the electrodes of the set of electrodes, to provide the physical contact between the container outer surface and the electrode, and the at least one movable element of the docking arrangement has a first position where at least one of the electrodes of the set of electrodes is detached from the outer surface of the container and a second position where the at least one electrode of the set of electrodes is held in physical contact with the outer surface of the container at an essentially constant pressure. The pressure between the set of electrodes and the outer surface of the container is maintained for a selected period of time.

In further embodiments of the present invention, the set of electrodes may be substantially plane and is preferably adapted to be in physical contact with a plane portion of the outer surface of a container, such as a bottom portion of the outer surface of a container. One of the electrodes of the set of electrodes may be circular, disc or ring shaped and one electrode of the set of electrodes may comprise a strip shaped electrode adapted for encircling and contacting an outer surface of the container, wherein the strip shaped electrode is associated to the docking arrangement, such that the strip shaped electrode is detached from the outer surface of the container when the docking arrangement is in a first position and is contacting the outer surface of the container when the docking arrangement is in a second position.

The docking arrangement in even further embodiments of the present invention comprises a spring element adapted, wherein said spring element is adapted to establish a pressure between at least one of the electrodes of the set of electrodes and an outer surface of the container, such as an outer surface of a bottom area of the container. The docking arrangement may comprise at least one constraining element adapted for holding the container in temporarily fixed condition, wherein the constraining element(s) preferably is adapted for holding the container in the temporarily fixed condition while the at least one movable element of the docking arrangement to establish a pressure between at least one of the electrodes of the set of electrodes and an outer surface of the container. The docking system may be adapted for electrochemical detection of growth of microorganisms in two or more containers, such as in two or more sub-containers of a multi container device, wherein the docking arrangement is adapted for temporarily holding said containers in location relative to respective sets of electrodes, to provide the respective electrode of the sets of electrodes to be in physical contact with an outer surface of said respective containers at respective preselected locations, wherein the electrodes of respective sets of electrodes are physically separated from each other.

In even further embodiments of the present invention, the system comprises a foil with a pressure sensitive adhesive adapted for being attached the outer surface of the container, preferably at a bottom location of the container and wherein an electrode of the set of electrodes are fixed on said foil.

In another aspect of the present invention, a method is provided for electrochemical detection of growth of microorganisms in a sample in a container. The method comprises docking the container in a docking system, providing the docking arrangement for temporarily holding the container in location relative to the set of electrodes, to provide the respective electrode to be in physical contact with an outer surface of said container at respective preselected locations, wherein the electrodes are physically separated from each other,, connecting the respective electrical connectors to an analyser; performing at least one electrical measurement and performing the detection of growth of microorganisms based on said at least one measurement.

Throughout the description or claims, the singular encompasses the plural and the plural encompasses the singular unless otherwise specified or required by the context.

**Brief description of the drawings**

   Figure 1 is a schematic illustration of an embodiment of the present invention of a docking system with non-contact electrodes adapted for real time detection of growth of microorganisms in a bottle container.

Figure 2 is a schematic illustration of another embodiment of the present invention of a docking system with non-contact electrodes adapted for real time detection of growth of microorganisms in a container.

Figure 3 is a schematic illustration of an embodiment of the present invention of a docking system with non-contact electrodes adapted for real time detection of growth of microorganisms in multiple containers of microwell plates.

Figure 4 is a schematic illustration of embodiments of the present invention of non-contact electrode configurations for real time detection of growth of microorganisms through an electrically insulated wall of a container.

Figure 5 is a schematic illustration of another embodiment of the present invention of non-contact electrode configurations for real time detection of growth of microorganisms through an electrically insulated wall of a container.

Figure 6 is a schematic illustration of a further embodiment of the present invention of a docking system with non-contact electrodes adapted for real time detection of growth of microorganisms in a laboratory flask.

Figure 7 is a schematic illustration of an embodiment of the method of the present invention of a docking system with non-contact electrodes adapted for real time detection of growth of microorganisms in microwell plates.

Figure 8 is a schematic illustration of an embodiment of the method of the present invention of a docking system with non-contact electrodes based on adapted for real time detection of growth of microorganisms in laboratory flasks.

Figure 9 is an example of an embodiment of the present invention with a plot of measured change in the real part of the impedance as measured at 10 kHz by an LCR meter on an embodiment as illustrated schematically in Figure 2.

Figure 10 is an example of an embodiment of the present invention with a plot of measured change in the imaginary part of the admittance as measured at 10 kHz by an LCR meter on an embodiment as illustrated schematically in Figure 2.

Figure 11 is an example of an embodiment of the present invention with a plot of measured change in the real part of the impedance as measured at 100 kHz by an LCR meter on an embodiment as illustrated schematically in Figure 2.

Figure 12 is an example of an embodiment of the present invention with a plot of measured change in the imaginary part of the admittance as measured at 100 kHz by an LCR meter on an embodiment as illustrated schematically in Figure 2.

Figure 13 is an example of an embodiment of the present invention with a plot of measured change in the real part of the impedance as measured at 10 MHz by an impedance analyser on an embodiment as illustrated schematically in Figure 5.

Figure 14 is an example of an embodiment of the present invention with a plot of measured change in the imaginary part of the admittance as measured at 10 MHz by an impedance analyser on an embodiment as illustrated schematically in Figure 5.

Figure 15 is an example of an embodiment of the present invention with a plot of measured change in the real part of the impedance as measured at 100 MHz by an impedance analyser on an embodiment as illustrated schematically in Figure 5.

Figure 16 is an example of an embodiment of the present invention with a plot of measured change in the imaginary part of the admittance as measured at 100 MHz by an impedance analyser on an embodiment as illustrated schematically in Figure 5.

**Detailed description of the invention**

The container may be, but is not limited to a bottle, a laboratory flask, a well of a microwell plate, a vial or the like. It may also be a tube or any hollow item, which contains a fluid or can be filled or partly filled with a fluid including a spacing between two plane plates. It may further be the top of a plane plate such as a microscope slide, where the surface tension of the liquid enables the liquid to be placed on the top surface of the plane plate; or it may be a concave plate or any item, which has the ability of containing a liquid. The container may be transparent, opaque or non-transparent for light. The present invention also covers arrays of containers, which may have essentially the same sizes and shapes, and arrays of containers with different sizes and shapes. The containers referred to in the description of the present invention comprise at least one electrically insulated wall through which the detection may be made. The insulated wall may be of a material, which is not electrically conductive, including but not limited to a glass, a polymer, an adhesive, or any combination comprising one or more of those materials.

In the following, "electrical connection" between two electrical elements, such as between an electrode of electrically conductive materials and an electrical connector should be understood as an ohmic or galvanic contact that allows electrical current to flow between two electrical elements, and with a reproducible and ideally negligible contact resistance and voltage drop. The tern "negligible" means that the contact resistance and associated voltage drop can be compensated for by an analyser, and that the contribution to the measurement uncertainty from the variations of the contact resistance and associated voltage drop is within acceptable limits. Acceptable limits mean within 10 %, more preferably within 1 % and even more preferably within 0.1 %. The actual value of how much contact resistance and associated voltage drop can be accepted depends on the impedance level, among others, and on the terminal configuration between the analyser used and the electrical connector. The "electrical connection" may be made by establishing a low electrical contact interface and/or a resistance between the two elements by soldering, bonding including ionic, covalent, and metallic bonding, gluing using electrically conductive glues or adhesives including but not limited to silver, gold, aluminium or the like; using electrical connectors with mechanical attachment, or by applying a sufficient mechanical force between surfaces of the two electrical elements, e.g. by means of a spring or the like. Other wordings used, which should be understood in the same sense, are "electrically connected" or simply "connected". The wording "A is connectable to B", where A and B are two items, means that a connection can be established between item A and item B.

In the following, the term "microorganisms" should be interpreted to comprise organisms such as bacteria, protozoa, algae, fungi, cells, and yeast or the like, covering but not exclusively organisms, which can be observed through an optical microscope or an electron microscope. The present invention covers but not exclusively electrochemical detection of growth of such microorganisms in a container.

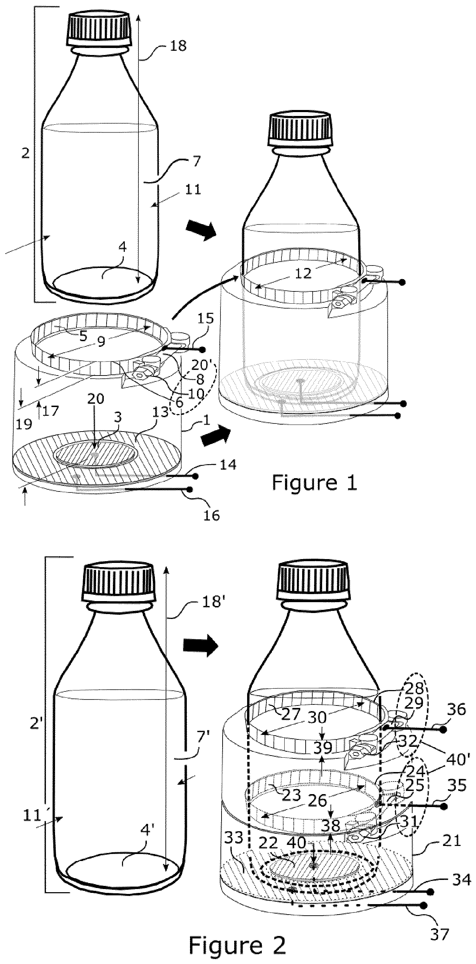

Figure 1 illustrates schematically, an embodiment of the present invention of a docking system with non-contact electrodes adapted for real time detection of growth of microorganisms through insulated walls of a container. The embodiment comprises a docking system with a docking station 1 adapted to dock a bottle 2, a docking site 20 preselecting the mounting position of the bottle and comprising a circular disc shaped first electrode of electrically conductive materials 3 being adapted to be attached to and detached from a preselected location on the outer surface of the bottom of a bottle 4, and a strip shaped second electrode of electrically conductive materials 5, and a docking arrangement 20' comprising a ring element 6 being adapted to be attached around a preselected location on the outer surface of a part of the cylindrical wall of the bottle 7 and to be detached from the preselected location on the outer surface of the part of the cylindrical wall of the bottle 7. The docking arrangement 20' further comprises an adjustment arrangement comprising a movable element 8 and a docking crew 10, which enable increasing and decreasing the diameter 9 of the ring element 6. The first electrode 3 and the second electrode 5 are one set of electrodes. In the embodiment as illustrated in Figure 1, the first electrode 3 is stationary fixed. The ring element 6 may have an annular shape, a shape as a ring a cylinder or the like.

At the position where the first electrode 3 is detached from the outer surface of the bottom of the bottle 4, and the second electrode 5 is detached from the outer surface of the cylindrical wall of the bottle 7, the docking arrangement is in the first position. At the position where the first electrode 3 is attached to a preselected location on the outer surface of the bottom of the bottle 4, and the second electrode is attached around a preselected location on the outer surface of a part of the cylindrical wall of the bottle 7, the docking arrangement is in the second position.

In the embodiment as illustrated in Figure 1, the docking station 1 is made with an inner diameter which is essentially the same or slightly larger than the outer diameter 11 of the bottle 2, and the ring element 6 is adapted to be attached to the docking station ensuring that the ring element 6 can be repositioned reproducible at a preselected location and with reproducible contact interfaces with minimized air spacing between the second electrode 5 and the part of the cylindrical outer wall of the bottle 7. The docking screw 10 enables loosening and tightening the ring element 6 around a part of the cylindrical outer wall of the bottle 7 and ensures tight attachment of the strip shaped second electrode 5 to the outer wall of the bottle 7.

Embodiments of the present invention include docking systems comprising docking stations and docking arrangements for attaching and detaching containers such as bottles or other types of labware to one or more sets of electrodes establishing the electrical connection. Such docking arrangements may consist of, but are not limited to spring loaded levers, pads, blades, and rollers, which exert pressure on the contact interfaces and ensure minimized air spacing between the sets of electrodes and the insulated walls of the containers. Embodiments of the present invention also include docking systems comprising docking arrangements for attaching and detaching containers controlled manually or by electrical or pneumatic actuators, pneumatic suction through pads, cups or the like, grommets of suitable shape and size, and electromagnets that keep ferrous materials attached to or being part of the containers. Embodiments of the present invention further include docking arrangements comprising clips or clamps, bayonet or threaded mounts, or screws with or without additional tension provided by means such as pneumatic, electrical actuators or operated manually; machined parts providing press-fit, or having slits, slots or other physical structures that allow containers to be positioned in the docking station with preselected positions of the electrodes on the surface of the outer wall of the container. In addition, the docking device may include elements to detect or adjust the positioning of the containers, or elements to check the electrical connections.

The bottle 2 can be detached from the docking station 1 by loosening the docking screw 10, and the diameter 9 of the ring element 6 becomes larger than the outer diameter of the cylindrical wall 11 of the bottle 2. The bottle 2 can be attached to the docking station 1 by tightening the docking screw 10, and the diameter 9 of the ring element 6 is reduced with an inner diameter 12 essentially equal to the outer diameter of the bottle 11. This may be made used a mechanical tool combined with or without a motorized drive. It may also be combined with a torque wrench to ensure that the tightening has the right torque and that the torque is reproduced between various mounting of bottles. An excessive torque may produce deformation or cracks in the wall of the bottle. A right torque means producing a suitable tightening pressure at the contact interface between the strip shaped second electrode 5 and the part of cylindrical outer wall of the glass to which the second electrode 5 is attached, and the air spacing between the second electrode 5 and the walls of the bottle 7 is minimized.

Gravity enables tight attachment of the circular disc shaped first electrode 3 to the preselected location at the surface of the outer wall bottom of the bottle 4. Alternatively, the docking screw 10 may be further adapted to enable a suitable tightening pressure at the docking site 20 onto the preselected location at the surface of the outer wall of the bottle to the first electrode 3. The first circular disc shaped electrode 3 may further comprise a planar surface or a curved surface adapted to minimize air spacing to the surface of the outer wall of the bottom of the bottle 4 or the circular disc shaped first electrode 3 may further comprise a combination of a thin conductive film on top of a flexible material such as a polymer foam combined with an adhesive such as a pressure sensitive adhesive. The circular disc shaped first electrode 3 may further comprise a spring element adapted for applying a mechanical force between the top surface of the first electrode 3 and the surface of the outer wall of the bottom of the bottle 4.

A guard ring of electrically conductive material 13 surrounds the first electrode 3 and minimizes stray electric fields. This is the preferred positioning of a guard ring. Alternatively, a guard may be positioned with parts above and below the strip shaped second electrode 5. An additional guard may be disposed around the docking station including covering the bottle. Design of guards with the purpose of reducing electrically stray fields is commonly used for capacitance sensors and it is well-known for a person skilled in the art.

The first electrode 3 is electrically connected or connectable to a first electrical connector 14 preferably made by a coaxial cable based on conductive material such as copper with the shield connected or connectable to the guard of the analyser used and the inner wire connected or connectable to the electrode 3 and with a suitable termination such as, but not limited to a BNC, SMA or SMB connector. The second electrode 5 is electrically connected or connectable to a second electrical connector 15 preferably made by a coaxial cable with the shield connected or connectable to the guard of the analyser used and the inner wire connected or connectable to the second electrical connector 15 and with a suitable termination such as, but not limited to a BNC, SMA or SMB connector. The first electrical connector 14 and the second electrical connector 15 are one set of electrical connectors. The guard ring 13 is electrically connected or connectable to a third electrical connector 16 with a suitable termination such as, but not limited to a BNC, SMA or SMB connector, which is preferably connected or connectable to the common earth or to the guard of the analyser used.

The set of electrodes 3 and 5 and the guard 13 may be made of a metal with high conductivity such as, but not limited to stainless steel, copper, aluminium, gold, silver or the like, or carbon-based conductive materials such as, but not limited to graphite, graphene, reduced graphene oxide-carbon or the like. Alternatively, the set of electrodes 3 and 5 and the guard 13 may be of conductive polymer materials such as, but not limited to polyacetylene, polyaniline, polythiophene, polypyrrole, polyfuran, poly(para-phenylene), and poly(phenylenevinylene).

For attachment of the second strip shaped electrode 5 around a preselected location on the surface of a part of the cylindrical outer wall of the bottle 7, a suitable tightening pressure is preferably in the range 10 $N/m^2$- 300 000 $N/m^2$, more preferably in the range 100 $N/m^2$ - 30 000 $N/m^2$, and even more preferably in the range 1 000 $N/m^2$ - 10 000 $N/m^2$. As it is well-known for a person skilled in the art, depending on the diameter of the ring element 12 and the height of the ring element 17, the suitable pressure can be converted to a suitable torque on the docking screw 10. The height of the second electrode 17 should preferably be in the range from 1/10 000 - 1/3 of the height of the bottle 18, more preferably be in the range from 3/1 000 - 1/8 of the height of the bottle 18, and even more preferably be in the range from 1/100 - 5/100 of the height of the bottle 18.

When docking a bottle 2 into the docking station 1 with the docking arrangement being in the second position, a preselected and reproducible tightening pressure is maintained temporarily by gravity and the ring element 6 between the disc shaped first electrode 3 and the outer surface of the bottom of the bottle 4, and between the strip shaped second electrode 5 and part of the cylindrical outer wall of the bottle 7, for a time period of at least 1 minute, such as at least 10 minutes. The time period should preferably be of the same order as or exceed the lag phase, the experimental growth phase and the stationary phase of bacterial growth.

There may be variations in the sizes and diameters of the bottles used with the present embodiment of the invention. The diameters of the docking station and the docking arrangement should advantageously be adapted to accept the variations in the diameters of the bottles used. The variation in the movable element 8 should advantageously be sufficiently wide to cover the tolerances or accept the variations in the outer diameters of the bottles used.

From the diameter of the first electrode 3, the height 17 and the diameter 9 of the second electrode 5 and the distance 19 between the first electrode 3 and the second electrode 5, a cell constant can be computed. Alternatively, the cell constant can be determined by measuring the impedance between the first electrical connector 14 and the second electrical connector 15 with the bottle 2 filled with a solution of a reference fluid of known electrolytic conductivity. As it is known by a person skilled in the art, the cell constant as determined between two electrodes multiplied by the capacitance as determined between the same two electrodes is equal to the effective dielectric constant, and determination of cell constant is equivalent to determination of capacitance.

The geometrical dimensions of the first electrode 3, the second electrode 5, the guard 13 and the distance between the first electrode and the second electrode 19 defining the cell constant should be adapted for real time detection of growth of microorganisms according to the electrolytic conductivity of the fluid inside the bottle 2 comprising the microorganisms to be detected. In "C. Thirstrup and L. Deleebeeck, IEEE Trans. Instrum. Meas. 70, 1008222 (2021)", it has been reviewed how to compute cell constants and how to determine cell constants using reference fluids. The contents and references therein are incorporated in the present invention.

Figure 2 illustrates another embodiment of the present invention. The embodiment comprises a docking system with a docking station 21 being adapted to dock a bottle 2', a docking site 40 preselecting the mounting position of the bottle and comprising a circular disc shaped first electrode 22 being adapted to be attached to and detached from a preselected location on the surface of the outer wall of the bottom of a bottle 4', a strip shaped second electrode 23, and a strip shaped third electrode 27 and a docking arrangement 40'. The docking arrangement 40' comprises a first ring element 24 being adapted to be attached around a preselected location on the surface of a first part of cylindrical outer wall 7' of the bottle 2' and to be detached from the preselected location on the surface of the part of the cylindrical outer wall 7' of the bottle 2'. The docking arrangement 40' further comprises a first adjustment arrangement comprising a first docking screw 31 and a first movable element 25, which enable increasing and decreasing the diameter 26 of the ring element 24. The first electrode 22 and the second electrode 23 constitutes a first set of electrodes. The docking arrangement 40' further comprises a second ring element 28 being adapted to be attached around a preselected location on the surface of a first part of cylindrical outer wall of the bottle 7' and to be detached from the preselected location on the surface of the part of the cylindrical outer wall of the bottle 7'. The docking arrangement 40' further comprises a second adjustment arrangement comprising a second docking screw 32 and a second movable element 29, which enable increasing and decreasing the diameter 30 of the second ring element 28. The first electrode 22 and the third electrode 27 constitutes a second set of electrodes. In the embodiment as illustrated in Figure 2, the first electrode 22 is stationary fixed.

At the position where the first electrode 22 is detached from the outer surface of the bottom of the bottle 4', the second electrode 23 is detached from the outer surface of the cylindrical wall of the bottle 7', the third electrode 27 is detached from the outer surface of the cylindrical wall of the bottle 7', the docking arrangement is in a first position. At the position where the first electrode 22 is attached to a preselected location on the outer surface of the bottom of the bottle 4, the second electrode 23 is attached around a preselected location on the outer surface of a part of the cylindrical wall of the bottle 7', and the third electrode 27 is attached around a preselected location on the outer surface of a part of the cylindrical wall of the bottle 7', the docking arrangement is in a second position.

In the embodiment as illustrated in Figure 2, the docking station 21 is made with an inner diameter which is essentially the same or slightly larger than the outer diameter 11' of the bottle 2'. The first ring element 24 is further being adapted to be attached to the docking station 21 ensuring that the first ring element 24 can be repositioned reproducibly at the preselected location and with reproducible contact interfaces with minimized air spacing between the second electrode 23 and a first part of the cylindrical outer wall of the bottle 7'; and the second ring element 28 is further being adapted to be attached to the docking station 21 ensuring that the second ring element 28 can be repositioned reproducible at the preselected location and with reproducible contact interfaces with minimized air spacing between the third electrode 27 and a second part of the cylindrical outer wall of the bottle 7'.

The first docking screw 31 enables loosening and tightening the first ring element 24 around the first part of the cylindrical outer wall 7' of the bottle 2' and ensures tight attachment of the strip shaped second electrode 23 to the first part of the cylindrical outer wall of the bottle 7'. The second docking screw 32 enables loosening and tightening the second ring element 28 around the second part of the cylindrical outer wall of the bottle 7' and ensures tight attachment of the second strip shaped electrode 27 to the second part of the cylindrical outer wall of the bottle 7'.

The bottle 2' may be detached from the docking station 21 by loosening the first docking screw 31, and the diameter 26 of the first ring element 24 becomes larger than the diameter of the cylindrical outer wall 11' of the bottle 2'; and by loosening the second docking screw 32, and the diameter 30 of the second ring element 28 becomes larger than the diameter of the cylindrical outer wall 11' of the bottle 2'. The bottle 2' can be attached to the docking station 21, by tightening the first docking screw 31, and the diameter 26 of the first ring element 24 is reduced to an inner diameter essentially equal to the outer diameter of the bottle 11'; and by tightening the second docking screw 32, and the diameter 30 of the second ring element 28 is reduced to an inner diameter essentially equal to the outer diameter of the bottle 11'. This may be made using a mechanical tool without or combined with a motorized drive. It may also be combined with the use of a torque wrench to ensure that the tightening of the first ring element 24 and the second ring element 28 have the right torques, and that the torques can be reproduced between various mounting of bottles. The phrase "right torques" mean herein producing a suitable tightening pressure at the contact interfaces between the strip shaped second electrode 23 and the first part of cylindrical outer wall of the bottle to which the second electrode 23 is attached, and between the strip shaped third electrode 27 and the second part of cylindrical outer wall of the bottle to which the third electrode 27 is attached; thereby providing that the air spacing between the second electrode 23 and the outer wall of the bottle 7 respectively between the third electrode 27 and the outer wall of the bottle 7 are minimized.

Gravity enables tight attachment of the circular disc shaped first electrode 22 to the preselected location at the surface of the outer wall of the bottom of the bottle 4'. Alternatively, the first docking screw 31 and/or the second docking screw 32 may be adapted to enable a suitable tightening pressure at the docking site 40 between the preselected location at the outer surface of the bottle and the circular disc shaped first electrode 22. The circular disc shaped first electrode 22 may further comprise a planar surface or a curved surface adapted to minimize air spacing to the surface of the outer wall of the bottom of the bottle 4', or the circular shaped first electrode 22 may further comprise a combination of a thin conductive film on top of a flexible material, such as a polymer foam combined with an adhesive such as a pressure sensitive adhesive. The circular disc shaped first electrode 22 may further comprise a spring element adapted for applying a mechanical force between the top surface of the first electrode 22 and the surface of the outer wall of the bottom of the bottle 4'.

A guard ring of electrically conductive materials 33 surrounds the first electrode 22 and minimizes stray electric fields. This is the preferred location of a guard ring. Alternatively, a first guard may comprise parts positioned above and below the second electrode 24, and a second guard may comprise parts positioned above and below the third electrode 27. An additional guard may be disposed around the docking station including covering the bottle 2'.

The embodiment as illustrated schematically in Figure 2 further comprises a first electrical connector 34 ensuring electrical connection to the first electrode 22 and with a suitable termination such as, but not limited to a BNC, SMA or SMB connector; a second electrical connector 35 ensuring electrical connection to the second electrode 23, and with a suitable termination such as, but not limited to a BNC, SMA or SMB connector; a third electrical connector 36 ensuring electrical connection to the third electrode 27 and with a suitable termination such as, but not limited to a BNC, SMA or SMB connector; and a fourth electrical connector 37 ensuring electrical connection to the guard 33 and with a suitable termination, such as, but not limited to a BNC, SMA or SMB connector, and which is preferably connected or connectable to the common earth or to the guard of the analyser used. The first electrical connector 34 and the second electrical connector 35 constitutes a first set of electrical connectors; and the first electrical connector 34 and the third electrical connector 36 constitutes a second set of electrical connectors.

The sets of electrodes 22, 23, 27 and the guard 33 may be made of a metal with high electrical conductivity such as, but not limited to stainless steel, copper, aluminium, gold, silver or the like, or carbon-based conductive materials such as, but not limited to graphite, graphene, reduced graphene oxide-carbon or the like. Alternatively, the sets of electrodes 22, 23, 27 and the guard 33 may be of conductive polymer materials such as but not limited to polyacetylene, polyaniline, polythiophene, polypyrrole, polyfuran, poly(para-phenylene), and poly(phenylenevinylene).

For attachment of the second electrode 23 around a first part of the cylindrical outer wall of a bottle 7' and attachment of the third electrode 27 around a second part of the cylindrical outer wall of the bottle 7', a suitable tightening pressure is preferably in the range 10 $N/m^2$ - 300 000 $N/m^2$, more preferably in the range 100 $N/m^2$ - 30 000 $N/m^2$, and even more preferably in the range 1 000 $N/m^2$ - 10 000 $N/m^2$. The height of the strip shaped second electrode 38 and the height of the strip shaped third electrode 39 should preferably be in the range from 1/10 000 - 1/3 of the height of the bottle 18', more preferably be in the range from 3/1000 - 1/8 of the height of the bottle 18', and even more preferably in the range from 1/100 - 5/100 of the height of the bottle 18'. The height 38 of the second electrode 24 and the height 39 of the third electrode 28 may be essentially identical or they may be different.

The cell constant as measured between the first electrical connector 34 and the second electrical connector 35 is smaller than the cell constant as measured between the first electrical connector 34 and the third electrical connector 36. A cell constant can also be measured from the second electrical connector 35 and the third electrical connector 36. The embodiment of the present invention as illustrated schematically in Figure 2 may be used in a differential measurement scheme, where impedance spectroscopy data recorded between the first electrical connector 34 and the second electrical connector 35, and impedance spectroscopy data recorded between the first electrical connector 34 and the third electrical connector 36 may be combined in order to eliminate optional contribution from the container walls to the data. The geometrical dimensions of the disc shaped first electrode 22, the strip shaped second electrode 23, the strip shaped third electrode 27, the guard 33 and the distance between each of the electrodes 22, 23 and 27 defining the cell constants should be adapted for real time detection of growth of microorganisms according to the electrolytic conductivity of the fluid inside the bottle 2' comprising the microorganisms to be detected.

Other embodiments of the present invention may include three, four or an even larger multitude of electrodes and a docking arrangement comprising a multitude of movable ring elements, each ring element being adapted to be attached around a preselected location on the outer surface of a part the cylindrical outer wall of the bottle 7', and to be detached from the preselected location on the outer surface of the part the cylindrical outer wall of the bottle 7'. The docking arrangement enables increasing and decreasing the diameter of each ring element in order for the multitude of electrodes to be attached to and detached from the bottle 2'.

The bottle 2 and 2' may be a standard bottle, e.g. from Schott Duran made of borosilicate glass, with volumes in the range from 5 mL - 10 L, where the wall thickness of the bottle typically is in the range from 1 mm to 3 mm, or the bottles 2 and 2' may be vials made of borosilicate glass or quartz with volumes in the range from 2 mL to 20 mL, where the wall thickness of the vial typically is 1 mm. The bottle material may also be other types of glass such as, but not limited to silicon dioxide, silicates, soda lime or aluminosilicate, or polymers such as, but not limited to low-density-polyethylene, high-density-polyethylene, polypropylene, polycarbonate, polystyrene and acrylics.

The present invention also includes embodiments, where a magnet is immersed in the bottle 2 or 2' or any other container, which combined with a magnetic stirrer ensures stirring of the fluid inside the container, and the first electrode 3 or 22 should preferably be made of a non-magnetically conductive material including, but not limited to aluminium, gold, silver, copper, titanium and zinc, non-magnetic stainless steel such as SUS305, alloys such as brass and bronze; conductive polymers and non-magnetic carbon compounds. The present invention also includes embodiments, where the fluid inside the container is mixed by an orbital shaker, an acoustic mixer or other types of mixers.

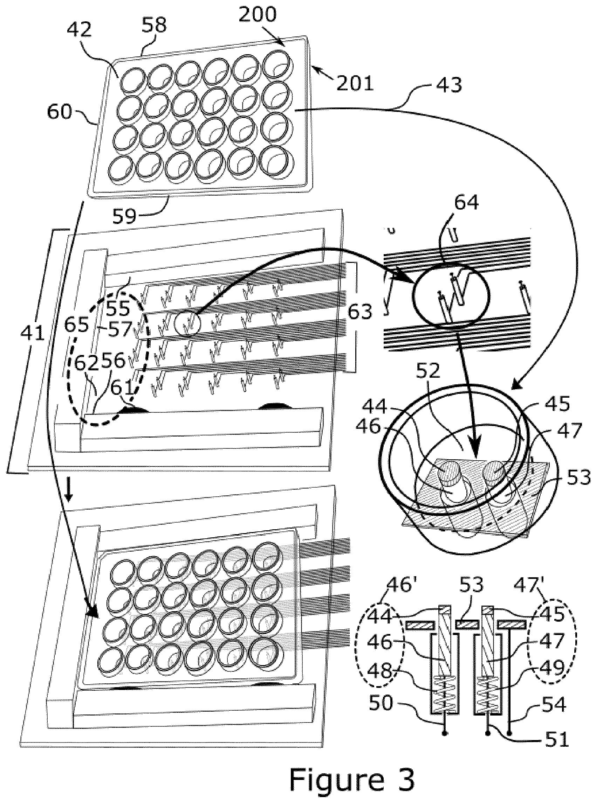

Figure 3 illustrates a further embodiment of the present invention of a docking system for real time detection of growth of microorganisms through insulated walls of containers or sub-containers. The embodiment comprises a docking system being adapted to dock a microwell plate 42 comprising a multitude of containers or sub-containers, also denoted wells 43. The top of the microwell plate 42 may comprise a cover plate 200, and the bottom of the microwell may comprise a plate 201, which may be a thin glass plate. The docking system comprises a docking station 41, one or more sets of a first pin element 46' and a first electrical connector 50 and a second pin element 47' and a second electrical connector 51. The first pin element 46' comprises a first electrode 44, a first movable and electrically conductive pin body 46, and a first spring element 48, where the first spring element 48 is engaged with the first electrode 44 via the first movable pin body 46. The second pin element 47' comprises a second electrode 45, a second movable and electrically conductive pin body 47, and a second spring element 49, where the second spring element 49 is engaged with the second electrode 45 via the second movable pin body 47. The first movable pin body 46 is adapted to attach and detach the contact interface of the first electrode 44 to and from the bottom insulated wall 52 of the container 43, and the second movable pin body 47 is adapted to attach and detach the contact interface of the second electrode 45 to and from the bottom insulated wall 52 of the container 43. The embodiment further comprises a guard 53 surrounding the first electrode 44 and the second electrode 45 minimizing stray electric fields. A second guard may be located at the top of the microwell plate ensuring that all containers of the microwell plate are electrically shielded. The guard 53 is connected or connectable to a third electrical connector 54. The first electrical connector 50, the second electrical connector 51 and the third electrical connector 50 each comprises a suitable termination such as, but not limited to a BNC, SMA or SMB connector. A multitude of electrical connectors (63) ensures electrical connection to each set of electrodes 44 and 45.

In the embodiment of the invention as illustrated in Figure 3, the docking system comprises a docking station 41 and a docking arrangement 65 of a plane surface 62, a first rail 55 being adapted to be attached to and detached from a first side of a microwell plate 58, a second rail 56 being adapted to be attached to and detached from a second side of a microwell plate 59, and a third rail 57 being adapted to be attached to and detached from a third side of a microwell plate 60, and a constraining element 61 ensuring that each set of first electrodes 44 and second electrodes 45 can be reproducibly attached to and detached from the docking sites 64 and with contact interfaces at preselected locations on the outer surfaces of insulated bottoms 52 of each container 43 of a microwell plate 42. Alternatively or additionally a force applying element, such as a solid bar, a plate, a lever or the like may be arranged to apply a force onto the top of the microwell plate 200 to insure a constant force between the bottom of the microwell plate on each set of first electrodes 44 and second electrodes 45. The force applying element may be of a conductive material and connected to ground and thereby shielding each set of first electrodes 44 and second electrodes 45 from external electromagnetic interference.

At the position where the first rail 55 is detached from the first side of the microwell plate 58, the second rail 56 is detached from the second side of the microwell plate 59, and the third rail 57 is detached from the third side of the microwell plate 60, the docking arrangement is in a first position. At the position where the first rail 55 is attached to the first side of the microwell plate 58, the second rail 56 is attached to the second side of the microwell plate 59, and the third rail 57 is attached to the third side of the microwell plate 60, the docking arrangement is in a second position.

In some embodiments of the present invention, the first rail 55 and the second rail 56 in combination with a constraining element 61 ensure sufficient force on the first side of a microwell plate 58 and the second side of a microwell plate 59 to reproducibly hold the microwell plate positioned with a sufficient pressure against each set of first electrode 44 and second electrode 45. The constraining element 61 may act as a spring and is preferably made from an elastic material such as a polymer or a metal such as stainless steel.

Other embodiments may comprise one or more elastic elements or spring elements being adapted on one or more rails ensuring reproducibly holding the microwell plate positioned with a sufficient pressure against each set of first electrode 44 and second electrode 45. When the microwell plate is docked, the bottom surfaces of each container 52 should preferably be in physical contact with the plane surface of the docking station 62. This can be provided by matching the height position of each first movable pin body 46 and each second movable pin body 47 with the plane surface of the docking station 62.

Each set of first movable pin element 46' and second movable pin element 47' may be mounted on a printed-circuit-board comprising a suitable layout of a multitude of electrical connectors ensuring electrical connection to each set of electrodes 44 and 45. The movable pin elements may be, but are not limited to two-part spring probes with a length in the range from 1 mm -200 mm, a diameter in the range from ø0.2 mm - ø5.0 mm and a head with a diameter in the range from ø0.05 mm - 20 mm, and a spring force in the range from 0.1 N - 100 N. The material may be, but is not limited to, a metal such as gold, silver, aluminium, or an alloy such as, but not limited to phosphor bronze. The selection includes an RS-PRO Rounded 2-part Spring probe with a length of 25 mm, a diameter of ø1.36 mm, a head of ø1.8 mm, a spring force of 1.47 N, and a contact resistance of 30 mΩ.

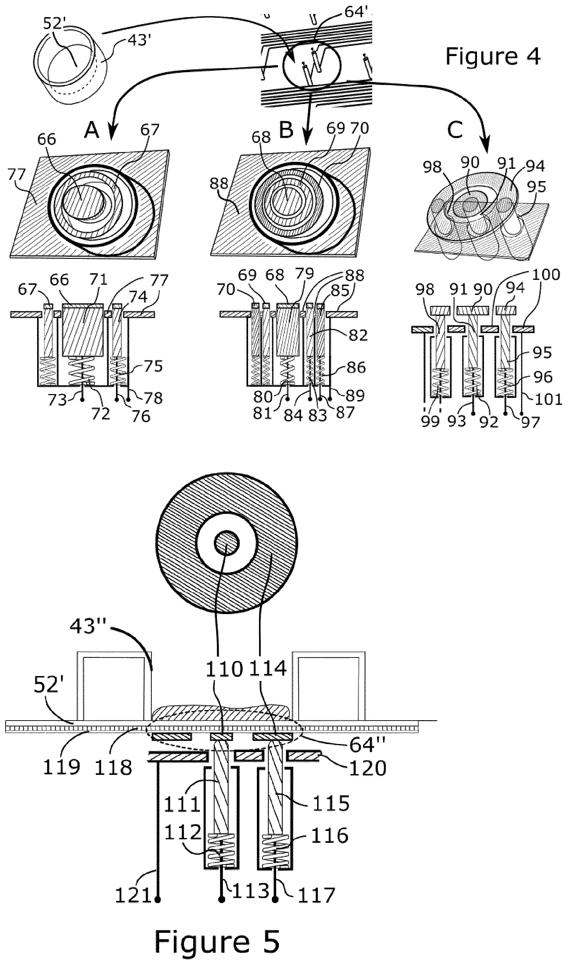

Figure 4 illustrates three other embodiments of sets of means for attachment and detachment of contact interfaces of sets of one or more electrodes at docking sites 64' with preselected locations on the outer surfaces of one or more insulated bottom plates of containers of microwell plates according to the embodiments of the present invention as illustrated in Figure 3.

Figure 4A illustrates attaching and detaching a set of electrodes according to further embodiments of the present invention comprising a first electrode 66 and a circular conductive rod with a plane or curved top-surface 71 and a first spring element 72 enabling the circular conductive rod 71 to move vertically, the disc shaped first electrode 66 having electrical connection to a first electrical connector 73; a cylindrical shaped second electrode 67, a circular conductive cylinder with a plane or curved top-surface 74 and a second spring element 75 enabling the circular conductive cylinder 74 to move vertically, the second electrode 67 having electrical connection to a second electrical connector 76, and a guard 77 surrounding the first electrode 66 and the second electrode 67 and having electrical connection to a third electrical connector 78. In the embodiment as illustrated in Figure 4A, the first spring element 72 is engaged with the first electrode 66 via the first circular conductive rod 71, and the second spring element 75 is engaged with the second electrode 67 via the circular conductive cylinder 74. The first electrical connector 73, the second electrical connector 76 and the third electrical connector 78 each comprises a suitable termination such as, but not limited to a BNC, SMA or SMB connector.

The disc shaped first electrode 66 is embedded at least partly inside the cylindrical shaped second electrode 67, and the inner diameter of the circular conductive cylinder 74 is larger than the outer diameter of the circular conductive rod 71. At least two of the electrical connectors must be physically separated and with essentially no direct electrical connection to each other. Part of the guard may be in between, but without physical contact to the first electrode 66 and the second electrode 67.

Figure 4B illustrates attaching and detaching a set of electrodes according to further embodiments of the present invention comprising a disc shaped first electrode 68 and a circular conductive rod with a plane top-surface 79 and a first spring element 80 enabling the first electrode 68 to move vertically, the first electrode 68 having electrical connection to a first electrical connector 81. The embodiment as illustrated in Figure 4B further comprises a cylindrical shaped second electrode 69 and a first circular conductive cylinder with a plane top-surface 82 and a second spring element 83 enabling the second electrode 69 to move vertically, the second electrode 69 having electrical connection to a second electrical connector 84. The embodiment as illustrated in Figure 4B further comprises a cylindrical shaped third electrode 70 and a second circular conductive cylinder with a plane top-surface 85 and a third spring element 86 enabling the third electrode 70 to move vertically, the third electrode 70 having electrical connection to a third electrical connector 87, and a guard 88 surrounding the first electrode 68, the second electrode 69 and the third electrode 70 and having electrical connection to a fourth electrical connector 89. In the embodiment as illustrated in Figure 4B, the first spring element 80 is engaged with the first electrode 68 via the first circular conductive rod 79; the second spring element 83 is engaged with the second electrode 69 via the first circular conductive cylinder 82;

and the third spring element 86 is engaged with the third electrode 70 via the second circular conductive cylinder 85.The first electrical connector 81, the second electrical connector 83, the third electrical connector 84 and the fourth electrical connector 89 each comprise a suitable termination such as, but not limited to a BNC, SMA or SMB connector.

The disc shaped first electrode 68 is embedded at least partly inside the cylindrical shaped second electrode 69, and the second electrode 69 is embedded at least partly inside the cylindrical shaped third electrode 70. The inner diameter of the first circular conductive cylinder 82 is larger than the outer diameter of the circular conductive rod 79 and the inner diameter of the second circular conductive cylinder 85 is larger than outer diameter of the first circular conductive cylinder 82. At least two of the electrical connectors must be physically separated and with essentially no direct electrical connection to each other. Part of the ground plane may be in between but without direct electrical connection to the first electrode 68, the second electrode 69 and the third electrode 70.

The present invention also includes embodiments with multitudes of circular conductive cylinders with increasing inner diameters, which are larger than the outer diameter of the circular conductive cylinders being embedded at least partly inside. The present invention also includes embodiments, where the circular conductive rod 71,79 is replaced by a circular conductive cylinder or other configurations such as, but not limited to a needle with a sharp tip, a rectangular rod or triangular rod. The present invention also includes embodiments where the circular conductive cylinders are replaced by rectangular conductive cylinders or cylinders with other geometrical shapes. The shapes and geometrical dimensions of the electrodes 66, 67, 68, 69 and 70 are adapted accordingly in these embodiments of the present invention.

Figure 4C illustrates attaching and detaching a set of electrodes according to further embodiments of the present invention, comprising a first electrode comprising a circular plate 90, a first movable and electrically conductive pin body 91, a spring 92 and a first electrical connector 93, and second electrode comprising a ring plate 94, a second movable and electrically conductive pin body 95, a spring element 96 and a second electrical connector 97 and a third movable pin body 98 comprising a spring element 99; the first movable pin body 91 being adapted to attach and detach contact interfaces of the first electrode 90 with the bottom insulated wall 52' of a container 43'; the second movable pin body 95 and the third movable pin body 98 being adapted to attach and detach contact interfaces of the second electrode 94 with the bottom insulated wall 52' of the container 43'. The embodiment further comprises a guard 100 surrounding the first electrode 90 and the second electrode 94 minimizing stray electric fields. A second guard may be positioned at the top of the microwell plate ensuring that all containers of the microwell plate are electrically shielded. The guard 100 is connected or connectable to a third electrical connector 101. The first electrical connector 93, the second electrical connector 97, and the third electrical connector 101 each comprise a suitable termination such as, but not limited to a BNC, SMA or SMB connector.

From the geometrical dimensions of the electrodes 66 and 67 and the guard 77 in Figure 4A and the distance between them, a cell constant can be determined or alternatively measured between the electrical connectors 73 and 76 using reference fluids. From knowledge the geometrical dimensions of the electrodes 79, 82, 85 and the guard 88 in Figure 4B, cell constants can be determined or alternatively measured between the electrical connectors 81 and 84, between 81 and 87 and between 84 and 87 using reference fluids, and the embodiment of the present invention as illustrated schematically in Figure 4B may be used in a differential measurement scheme, where impedance spectroscopy data recorded between the first electrical connector 81 and the second electrical connector 84 and impedance spectroscopy data recorded between the first electrical connector 81 and the third electrical connector 87 may be combined in order to eliminate the contribution from the container walls to the data. From the geometrical dimensions of the electrodes 90, 94 and the guard 100 in Figure 4C and the distance between them, a cell constant can be determined or alternatively measured between the electrical connectors 93 and 97 using reference fluids.

The geometrical dimensions of the electrodes 66 and 67 and the guard 77 in Figure 4A, the electrodes 68, 69, 70 and the guard 88 in Figure 4B, and the electrodes 90, 94 and the guard 100 in Figure 4C, and the distance between them defining cell constants may in each pairwise combination be adapted for real time detection of growth of microorganisms according to the electrolytic conductivity of the fluid inside the container 43' comprising the microorganisms to be detected.

The present invention also includes embodiments with sets of multitudes of electrodes comprising circular plates and ring plates with increasing inner diameters which are larger than the outer diameter of the circular conductive plates being embedded at least partly inside. The present invention also includes embodiments, where the first electrodes as circular conductive discs 66 and 68 are replaced by circular conductive rings or other configurations like but not limited to a needle with a sharp tip, a rectangular plate or triangular plate. The present invention also includes embodiments where the circular conductive ring plates 67, 68 and 69 are replaced by rectangular conductive ring plates or plates with other geometrical shapes.

Figure 5 illustrates attaching and detaching a set of electrodes to and from contact interfaces at preselected locations on the outer surfaces of one or more insulated bottom plates of containers of microwell plates according to even further embodiments of the present invention. The embodiment in Figure 5 comprises a disc shaped first electrode 110, a first movable and electrically conductive pin body 111, a spring element 112 and a first electrical connector 113, a ring shaped second electrode 114, a second movable and electrically conductive pin body 115, a spring element 116 and a second electrical connector 117. The first spring element 112 is engaged with first electrode 110 via the first movable pin body 111, and the second spring element 116 is engaged with second electrode 114 via the second movable pin body 115. The embodiment as illustrated in Figure 5 further comprises an adhesive element 118 adapted to be attached to the bottom insulated wall 52" of a container 43" and comprising a non-electrically-conductive foil 119, and where the first electrode 110 and the second electrode 114 are disposed on the side of the foil 119 not adhering to the adhesive element 118. After attachment, the adhesive element 118 may be further adapted to be detached from the bottom insulated wall 52" of a container 43". At docking sites 64", the first movable pin body 111 is adapted to attach and detach the electrical connection between the first electrical connector 113 and the contact interface of the first electrode 110 with the bottom insulated wall 52" of the container 43"; and the second movable pin body 115 is adapted to attach and detach the electrical connection between the second electrical connector 117 and the contact interface of the second electrode 114 with the bottom insulated wall 52" of the container 43". The embodiment further comprises a guard 120 surrounding the first movable pin body 111 and the second movable pin body 115 minimizing stray electric fields. A second guard may be positioned at the top of the microwell plate ensuring that all containers of the microwell plate are electrically shielded. The guard 120 is connected or connectable to a third electrical connector 121. The first electrical connector 113, the second electrical connector 117, and the third electrical connector 121 each comprise a suitable termination such as, but not limited to a BNC, SMA or SMB connector.

From the geometrical dimensions of the electrodes 110 and 114 and the guard 120 in Figure 5 and the distance between them, a cell constant can be determined or alternatively measured between the electrical connectors 113 and 117 using reference fluids. The geometrical dimensions of the electrodes 110 and 114 in Figure 5 may conveniently be adapted for real time detection of growth of microorganisms according to the electrolytic conductivity of the fluid inside the container 43" comprising the microorganisms to be detected.

The present invention also includes embodiments with sets of multitudes of electrodes disposed on one or more foils on top of adhesive elements, the electrodes comprising circular plates and ring plates with increasing inner diameters which are larger than the outer diameter of the circular conductive plates being embedded at least partly inside. The present invention also includes embodiments, where the circular conductive disc 110 is replaced by a circular conductive ring or other configurations like but not limited to a needle with a sharp tip, a rectangular plate or triangular plate. The present invention also includes embodiments where the circular conductive ring plates 114 are replaced by rectangular conductive ring plates or plates with other geometrical shapes.

The present invention also includes embodiments where connections are made through printed circuit boards. These boards may also include switching circuitry, electronic circuitry for the impedance analysis and other circuitries to facilitate the docking, adjustments of the docking, calibration, compensation, guarding, or other purposes.

The adhesive element 118 may be a pressure sensitive adhesive based on materials such as but not limited to polyacrylates and silicone rubber and may comprise removable adhesives designed to repeatedly stick and unstick, and the foil 119 may be a polymer foil made of a polymer such as but not limited to polypropylene, polyvinyl chloride and polyethylene. The adhesive may be equipped with a release liner, which is removed prior to disposing the adhesive to the bottom plate of the microwell plate. The electrodes 110 and 114 may be screen printed or tampo printed onto the foil 119, where tampo printed is also known as pad printing and tampography, and may be of a conductive film of a material such as but not limited to silver, aluminium and gold.

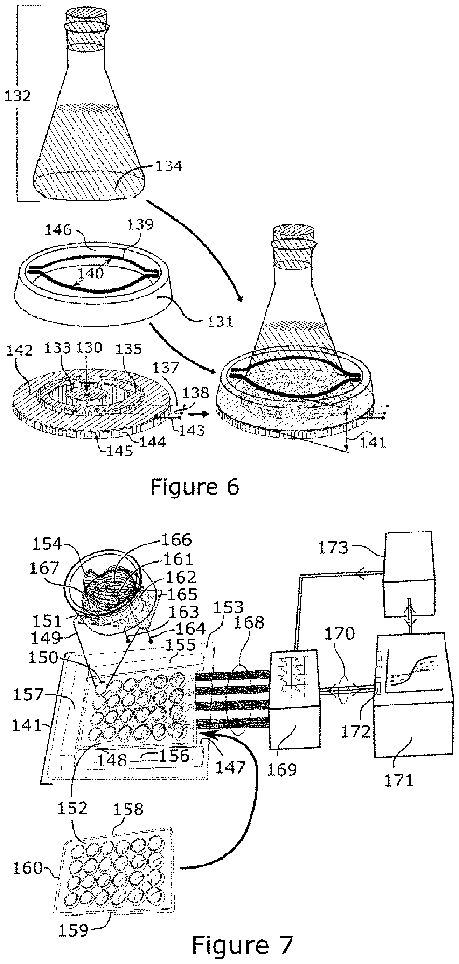

Figure 6 illustrates schematically, an even further embodiment of the present invention of a docking system with non-contact electrodes adapted for real time detection of growth of microorganisms through insulated walls of a container such as but not limited to a laboratory flask or an Erlenmeyer flask.

The embodiment comprises a docking system with a docking station 131 adapted to dock a flask 132, a circular disc shaped first electrode 133 being adapted to be attached to and detached from a preselected location on the outer surface of the bottom of the flask 134 and with a connection to a first electrical connector 137. The embodiment in Figure 6 further comprises a circular ring shaped second electrode 135 being adapted to be attached to and detached from a preselected location on the outer surface of the bottom wall of the flask 134 and with electrical connection to a second electrical connector 138. The docking system comprises a docking station 131 and a docking arrangement with a spring element of elastic material 139, which is able to move and thereby change diameter, enabling the flask 132 to be attached to and detached from the docking station 131 at a docking site 130; the spring element 139 being adapted to ensure tight attachment of the first electrode 131 and the second electrode 135 to the outer surface of the bottom wall of the flask 134; and the spring element 139 further being adapted to ensure that the flask can be repositioned reproducible at the docking site 130 with a preselected location on the outer surface of the bottom wall of the flask when attaching and detaching the flask 132 to and from the docking station 131. The spring element 139 is adapted to have an inner diameter 140 which can move between being smaller or larger than the diameter of the flask at the height 141, where the spring element 139 is in contact with the side outer wall of the flask 132.

In an embodiment, the first electrode 133 and/or the second electrode 135 may further comprise one or more spring elements applying a mechanical force between the top surfaces of the first electrode 133 and the second electrode 135 and outer surface of the bottom wall of the flask 134.

At the position where the first electrode 131 and the second electrode 135 are detached from the outer surface of the bottom wall of the flask 134, the docking arrangement is in a first position. At the position where the first electrode 131 and the second electrode 135 are attached to preselected locations on the outer surface of the bottom wall of the flask 134, the docking arrangement is in a second position.

The embodiment of the present invention as illustrated in Figure 6 further comprises a guard element 142 being adapted to shield the first electrode 133 and the second electrode 135 and with electrical connection to a third electrical connector 143, and it may further comprise an adhesive element 144 and a foil 145, where the first electrode 133, the second electrode 135 and the guard 142 are disposed on top of the foil 145. The electrodes 133 and 135 and the guard 142 may be screen printed or tampo printed onto the foil 145 and may be of a conductive film of a material such as but not limited to silver, aluminium, copper and gold.

The present invention also comprises embodiments where the attachment and detachment of the first electrode 133 and the second electrode 135 to the bottom outer wall of a flask 134 are established fully or partly by means of gravity. The present invention also comprises embodiments, where the inner wall of the docking station 146 is adapted to work as a spring element ensuring tight attachment of the first electrode 133 and the second electrode 135 to the bottom outer wall of the flask 134, and where the docking station 131 is further being adapted to ensure that the flask may be repositioned reproducible at a docking site 130 with a preselected location on the outer surface of the bottom wall of the flask when detaching and attaching the flask 132 from the docking station 131.

The present invention also includes embodiments where the electrodes 133 and 135 are combined with multitudes of electrodes comprising circular plates and ring plates with increasing inner diameters, which are larger than the outer diameter of the circular conductive plates being embedded at least partly inside. The present invention also includes embodiments, where the circular conductive disc 133 is replaced by a circular conductive ring or other configurations like, but not limited to a needle with a sharp tip, a rectangular plate or triangular plate. The present invention also includes embodiments where the circular conductive ring plates including 135 are replaced by rectangular conductive ring plates or plates with annular or other geometrical shapes. The present invention further includes embodiments where the spring element 139 is engaged with an additional strip shaped electrode. Laboratory flasks may have, but are not limited to have volumes in the range from 50 mL to 3 L with a wall thickness typically in the range from 1 mm to 3 mm, but not limited to this range, and be made of a glass such as but not limited to borosilicate or soda lime, or plastics such as but not limited to polypropylene, polymethylpentene, polycarbonate or polytetrafluoroetylen. There may be variations in the sizes and diameters of the flasks used with the present embodiment of the invention. The inner wall of the docking station 146 and the diameter 140 of the spring element 139 should be designed in order to accept the tolerances or the variations in the diameters of the flasks used.

From the geometrical dimensions of the electrodes 133 and 135 in Figure 6 and the distance between them, a cell constant can be determined or alternatively measured between the electrical connectors 137 and 138 using reference fluids.

The adhesive element 144 may be a pressure sensitive adhesive based on materials such as but not limited to polyacrylates and silicone rubber and may comprise removable adhesives designed to repeatedly stick and unstick, and the foil 145 may be a polymer foil made of a polymer such as but not limited to polypropylene, polyvinyl chloride and polyethylene. The adhesive element 144 may be equipped with a release liner, which is removed prior to disposing the adhesive below the docking station 131.

Figure 7 illustrates another aspect of the present invention with an embodiment of a non-contact electrode measurement method for real time detection of growth of microorganisms through electrically insulated walls 151 of containers or sub-containers 150 comprising the steps of: docking a microwell plate 152 comprising a multitude of containers, also denoted wells 150 to a docking station 153, each container comprising the sample of microorganisms 154 to be analysed. The docking station 153 comprises a docking arrangement comprising a plane surface 147, a first rail 155 being adapted to be attached to a first side of a microwell plate 158, a second rail 156 being adapted to be attached to a second side of a microwell plate 159, and a third rail 157 being adapted to be attached to a third side of a microwell plate 160, and a constraining element 148 ensuring that each set of first electrode 166 and second electrode 167 can be reproducibly detached from and attached to docking sites 149 with contact interfaces at preselected locations on the outer surfaces of each insulated bottom 151 of each container 150 of the microwell plate 152.

In some embodiments of the present invention, the first rail 155 and the second rail 156 in combination with the constraining element 148 ensure sufficient force on the first side of a microwell plate 158 and the second side of a microwell plate 159 to reproducibly hold the microwell plate positioned with a sufficient pressure against each set of first electrode 161 and second electrode 166. The constraining element 148 may act as a spring and is preferably at least partly made from an elastic material such as a polymer or a metal such as stainless steel.

At each container 150, by means of a first movable pin body 161 and a second movable pin body 162, a set of electrical connectors 163 and 164 physically separated from each other and shielded by a guard 165 are brought in electrical connection via a physically separated set of electrodes 161 and 166 to the insulated wall 151 of the container 150. Each set of one or more electrical connectors 163, 164 used for the microwell plate comprise a suitable termination such as, but not limited to a BNC, SMA or SMB connector.

Each set of one or more electrical connectors may be electrically connected or connectable through a first set of cables 168 to an electrical switch 169, and the switch is further electrically connected or connectable to an analyser 171 with one or more sets of channels 172 through a second set of cables 170; the electrical switch 169 being adapted to direct electrical signals from a guard 165 and from the one or more sets of electrodes 166 and 167 both having contact interfaces with the outer surfaces of insulated walls 151 of each container 150 to a set of channels 172 of the analyser 171, which derives impedance data from the microorganisms in each container and displays and/or stores the data as real time detection of growth of microorganisms in a memory of a computer or the like 173.

The analyser may be an electrochemical analyser such as an instrument making measurements of impedance parameters, like impedance magnitude and phase as a function of frequency, or components values, such as but not limited to inductance (L), capacitance (C) and resistance (R), as a function of frequency. The analyser may be, but is not limited to a commercial impedance analyser, LCR meter, vector impedance meter, LCZ meter, or a network analyser with suitable software that allows derivation of impedance features, or an instrument made from generators, digitizers and appropriate signal conditioning devices and software to calculate impedance properties, or a customized instrument made from suitable components and with software to calculate impedance properties. The connector topology of the analyser includes, but is not limited to common 4-terminal, 2-terminal or 1-terminal configurations. The instrument may apply a single tone signal, a swept signal, or a multitude of signals. The instrument may also be an instrument which operates in the time domain by applying a signal including but not limited to pulses or pulse trains, ramps, or composite signals, and which derives impedance properties through data processing. The analyser may be separate from the docking system; it may be built into the docking system, or parts of the analyser may be built into the docking system.

One embodiment of the present invention with a docking system comprising a docking station and multiple sets of electrodes adapted to analyse a sample with growth of microorganisms in multiple containers may comprise one analyser and a switching network. Another embodiment of the present invention of a docking system with a docking system comprising a docking station and multiple sets of electrodes may comprise one analyser adapted for analysis of each container. A further embodiment of the present invention may comprise multiple analysers, but fewer than the number of containers, and a switching network. The docking system, the containers and the switching network may include shielding or guarding, active or passive. The docking system may include components to calibrate the instrument and components for compensation of parasitic capacitance, like stray capacitance, unwanted inductive coupling, series inductance, series resistance or parallel conductance. The switching network may comprise an array of mechanical relays, semiconductor relays or switches, or a combination of these.

The analyser may also be a 2-terminal network analyser, which measures the electrical complex transfer function of the sample located in a container in shape of a bottle or other labware and electrically connected or connectable through the docking system to the 2 terminals of the network analyser. The electrical complex transfer function includes, but is not limited to measures of transfer magnitude and phase, or transfer scattering parameters, known as S-parameters, S12 or S21. The impedance properties of the sample in the container may be derived from the electrical complex transfer function by use of software.

The analyser may also be a 1-terminal network analyser or impedance meter, which measures the electrical complex reflection function or the impedance of the sample located in a container in shape of a bottle or other types of labware and electrically connected or connectable through the docking system to the terminal of the analyser. The electrical complex reflection function includes, but is not limited to measurements of reflection scattering parameters, known as the S-parameters, S11 or S22. The properties of the sample in the container may be derived from the electrical complex reflection function or impedance by use of software.

In addition to common scattering parameters (S-parameters) the analyser may also exploit X-parameters, which are a generalization of the linear S-parameters to characterize non-linear components and systems, and any other parameters that may be applied to characterize any non-linear properties of the sample in the container.

In one embodiment of the present invention, impedance matching is included in order to obtain larger or smaller voltages or currents of excitation or detection, or to improve the impedance matching, or for other purposes. An impedance matching may be integrated into the docking system, or may be disposed outside the docking system.

The electrical switch may comprise an array of mechanical relays with low contact resistance and low capacitance such as but not limited to the Pickering Series 103, Low Capacitance SIL/SIP Reed Relays. Alternatively, the electrical switch may comprise an array of solid-state relays or other types of relays or switches. The analyser may be an instrument making measurements in the frequency domain such as an LCR meter measuring inductance (L), capacitance (C) and resistance (R), including but not limited to a Keysight Precision LCR meter (20 Hz - 2 MHz) E4980A, a Keysight E4990A impedance analyser with a frequency range from 20 Hz to 120 MHz or a Wayne Kerr 65120B Precision Impedance Analysers operating at frequencies from 20 Hz to 120 MHz; or it may be a vector network analyser such as, but not limited to a Rohde & Schwarz ZNB20 operating at frequencies from 100 kHz to 20 GHz, or other types of instruments operating in the frequency domain. When making analysis above several MHz and in particular in the GHz regime, it becomes increasingly important to match the impedance between the electrical connectors 163 and 163 with the characteristic impedance of the analyser, typically 50 $\Omega$. A person skilled in the art knows how to optimize the impedance matching.

The method of the present invention also includes steps of making open-circuit measurements, short-circuit measurements, load measurements and when required at low conductivities, low-loss capacitor measurements prior to making analysis of growth of microorganisms. Open-circuit measurements are made with an open-circuit-component docked into the docking station 153 and making impedance measurements at each preselected frequency or over the frequency range used by the analyser 171 for each set of one or more electrical connectors 163, 164 used for the microwell plate, and with the corresponding positions of the relays in the electrical switch 169. The open-circuit-component may be a microwell plate identical to or of the same type as the one used in analysis of a sample with growth of microorganisms, where each well or container is empty or filled with a gas such as air, or where each well or container is filled with a calibration fluid such as DI water, a solution of a salt such as KCl or a fluid such as a growth medium including, but not limited to BHI media. Short-circuit measurements are made with a short-circuit-component, by short circuiting each set of one or more electrical connectors 163, 164 used for the microwell plate and the guard, and making impedance measurements at each predefined frequency or over the frequency range used by the analyser. The short-circuit-component exhibits a high electric conductivity and may comprise conductive plates or wires of metals such as, but not limited to copper, gold, silver, conductive polymer films, and carbon-based films. The open-circuit measurements should preferably be made with each set of first movable pin body 161 and second movable pin body 162 essentially be at the same preselected position as when the analyses are made on a microwell plate 152 docked into the docking station 153 and with the open-circuit-component be positioned at the predefined position. The short-circuit measurements should preferably be made with each set of a first movable pin body 161 and a second movable pin body 162 essentially be at the same predefined position as when analyses are made on a microwell plate 152 docked in the docking station 153 and with the short-circuit-component be positioned at that preselected position.

Load measurements are made with a load-component having a known impedance, by connecting the load-component to each set of one or more electrical connectors 163, 164 used for the microwell plate and the guard, and making impedance measurements at each predefined frequency or over the frequency range used by the analyser. The load-component exhibits a well-known impedance or electric conductivity and may comprise passive and/or active components. Preferably, the value of the load should be within the measurement range of analyser. In case the analyser has multiple measurement ranges, different values of load can be used, and measurements are carried out for each range to establish traceability. A person skilled in the art knows how to select the load impedance values and to carry out load measurements. The load measurements should preferably be made with each set of first movable pin body 161 and second movable pin body 162 essentially be at the same preselected position as when the analyses are made on a microwell plate 152 docked into the docking station 153 and with the load-component be positioned at the predefined position.

The method of the present invention also includes steps of modelling the impedance data by equivalent electrical circuits. A number of models have been reported in the scientific literature, including [C.E. Turick et al. Appl. Microbiol. Biotechnol. 103, 8327-8338 (2019)], [R. Gnaim et al. BioTechniques 69, 27-36 (2020)], [A. L. A. de Araujo et al. Biosensors 9, 108, 1-4 (2019)] and [M. Simic et al. IEEE Sensors J. 20, 12791-12797 (2020)]. The equivalent electrical circuits modelling the embodiments of the present invention include a capacitor related to the electrically insulated walls of containers and should be included in the equivalent electrical circuit as a parallel capacitor. The modelling may determine the species of the microorganisms and discriminate dead microorganisms from live microorganisms.

Simple circuit models may be expressed in terms of parallel admittance ($Y_p$), where a conductance ($G_p$ or the reciprocal resistance, $R_p$) and a susceptance ($B_p$ or the reciprocal reactance, $X_p$) are connected in parallel. Alternatively, simple circuit models may be or expressed in terms of series impedance ($Z_s$), where a resistor ($R_s$) and a reactance ($X_s$) are connected in series. There are several other combinations of making circuit models, and it is simple to transform one circuit model into another type of circuit model. The relationship between ($R_p$, $B_p$) and ($R_s$, $X_s$) is;

$$R_s = \frac{R_p}{1+\left(B_p R_p\right)^2} \; ; \; -X_s = R_s B_p R_p.$$

The method of the present invention also includes steps relating the impedance data as detected by the analyser 171 to a number of microorganisms, which includes taking subsamples from the sample of microorganisms 154 and count the number of microorganisms using standard methods such as colony-forming-units (CFU), optical density measurements at 600 nm ($OD_{600}$) using calibrated instruments, or using similar methods. A person skilled in the art knows how to make such calibration measurements. Making open-circuit measurements and short-circuit measurements subtracts the effect of the electrically insulated walls of a container, which can be considered to be a parallel capacitor. In combination with reproducible positioning of each container used of a microwell plate at essentially the same preselected location when attaching and detaching the microwell plate ensures reproducible measurements and enable absolute detection of the number of microorganisms in real time.

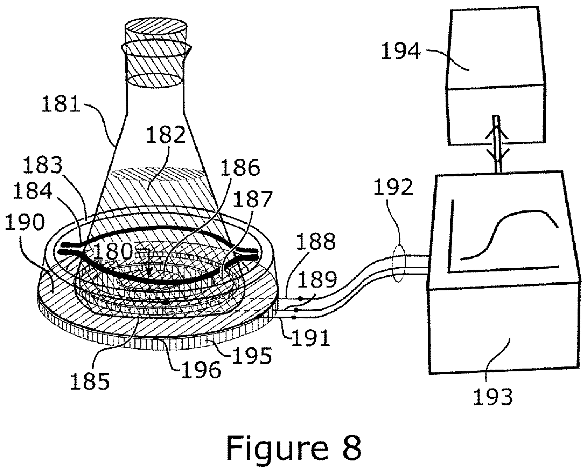

Figure 8 illustrates another embodiment of the present invention of a non-contact electrode measurement method for real time detection of growth of microorganisms through electrically insulated walls of containers comprising the steps of: docking a flask 181 comprising the sample of microorganisms 182 to be analysed by docking the flask into a docking system with a docking station 183 and a docking arrangement comprising a spring element of elastic material 184, which is able to move and thereby change diameter. This enables the flask 181 to be attached to and detached from the docking station 183 at a docking site 180 and it ensures tight attachment to the outer surface of the bottom wall of the flask 185 of the first electrode 186 with an electrical connection to a first electrical connector 188, and the second electrode 187 with an electrical connection to a second electrical connector 189, and with a guard 190 having electrical connection to a third electrical connector 191. The spring element 184 is further being adapted to ensure that the flask 181 can be repositioned reproducible at the docking site 180 with a preselected location on the outer surface of the bottom wall of the flask 181 when attaching and detaching the flask 181 to and from the docking station 183.

In an embodiment, the first electrode 186 and/or the second electrode 187 may further comprise one or more spring elements applying a mechanical force between the top surfaces of the first electrode 186 and the second electrode 187 and the outer surface of the bottom wall of the flask 185.

The first, the second and the third electrical connectors 188, 189 and 191 are electrically connected or connectable through a set of cables 192 to an analyser 193 by directing electrical signals from the guard 190, each set of first electrode 186 and second electrode 187 having contact interfaces with the insulated outer surface of the bottom wall of the flask 185 to the analyser 193, which derives impedance data from the microorganisms in the flask and displays and/or stores the data of real time detection of growth of microorganisms in a memory of a computer or the like 194.

The docking system may further comprise an adhesive element 195 and a foil 196, where the first electrode 186, the second electrode 187 and the guard 190 are disposed on top of the foil 196. The electrodes 186 and 187 may be screen printed or tampo printed onto the foil 196 and may be of a conductive film of a material such as, but not limited to silver, aluminium, copper of gold. The present method of the invention may also include attaching the adhesive element 195, the other parts of the docking system and the flask 181 in an orbital shaker in order to ensure effective stirring of the fluid comprising the microorganisms.

The method of the present invention also includes steps of making open-circuit measurements and short-circuits measurements prior to making analysis of growth of microorganisms. Open-circuit measurements are made with an open-circuit-component docked into the docking station 183 and making impedance measurements at each preselected frequency or over the frequency range used by the analyser between the electrical connectors 188 and 189. The open-circuit-component may be a flask identical to or of the same type as the one used in analyses of growth of microorganisms, where the flask is empty or filled with a gas such as air, or where the flask is filled with a calibration fluid such as water, a solution of a salt such as KCl, or a fluid such as a growth medium including but not limited to BHI media.

In combination with reproducibly docking the container into a docking station with attachment and detachment of the containers at preselected locations of electrodes on the outer surface of one or more containers and with reproducible tightening pressure at the contact interfaces between the electrodes and the outer walls of the containers, the open-circuit-measurement enables elimination of individual variations in container wall thickness, variation in container wall material composition, wall roughness and other materials and structural parameters.

Short-circuit measurements are made with a short-circuit-component by short circuiting the electrical connectors 188 and 189 and the electrical connector for the guard 191 and making impedance measurements at each preselected frequency or over the frequency range used by the analyser. The short-circuit-component exhibits high electric conductivity and comprises one or more conductive plates or wires of conductive materials such as, but not limited to copper, gold, silver, conductive polymer films, carbon-based films or the like. The open-circuit measurements should preferably be made with the first electrode 186 and the second electrode 187 essentially at the same preselected location as when the analyses are made on a flask 181 docked into the docking station 183 at the docking site 180 and with the open-circuit-component be positioned at that preselected location. The short-circuit measurements should preferably be made with the first electrode 186 and the second electrode 187 essentially be at the same predefined positions as when the analyses are made on a flask 181 docked into the docking station 183 at the docking site 180 and with the short-circuit-component be positioned at the preselected location.

Open-circuit measurements and short-circuit measurements are procedures, which are normally integrated in analysers such as LCR-meters and impedance analysers. For an analyser with no integration of such procedures, a person skilled in the art knows how to correct the impedance data recorded with the analyser with respect to the open-circuit measurements, the short-circuit measurements, and the load measurements.

The method of the present invention also includes analysers based on measurements in the time domain short as methods based on applying current pulses and measuring the voltage response, or applying voltage pulses and measuring the current response. The width of the pulses, repetition rates and duty cycles used for the analysers making measurements in the time domain can be optimized according to knowledge of suitable frequencies used with analysers making measurements in the frequency domain and using mathematical tools such as Laplace transforms or inverse Laplace transforms, which relate the frequency domain and the time domain. This is described in the literature, e.g. in "C. Thirstrup and L. Deleebeeck, IEEE Trans Instrum Meas 70, 1008222 (2021)".

Example 1

A docking system with non-contact electrodes adapted for real time detection of growth of microorganisms through insulated walls of a container as illustrated schematically in Figure 2 adapted for 500 mL glass bottles (Schott DURAN borosilicate glass bottles, 500 mL, ø85 mm) was used in combination with an Agilent E4980A LCR meter combined with a computer and software as analyser. In the docking system, the docking station 21 was fabricated of polymer materials by 3D printing, a circular disc shaped first electrode 22 was made of a copper plate with a thickness of 0.5 mm and diameter of 40.0 mm. The guard 33 was made of a copper plate with a thickness of 0.5 mm, an inner a diameter of 40.0 mm and an outer diameter of 85.0 mm, and it was positioned 1 mm lower than the first electrode 22. A strip shaped second electrode 23, and a strip shaped third electrode 27, were each made of stainless steel with a height of 12 mm. A docking arrangement comprised a first ring element 24 with a height of 12 mm, a first movable element 25, a second ring element 28 with a height of 12 mm, a second movable element 29 and docking screws 31 and 32, each element was made of stainless steel, enabled changing the diameter of the ring elements from 80 mm to 100 mm. The distance as defined in Figure 2 between the first electrode 22 and the second electrode 23 was 35 mm, and similarly the distance between the first electrode 22 and the third electrode 27 was 85 mm. The electrical connectors 34 and 37 were fabricated by soldering the inner wires of coaxial cables to the bottoms of the first electrode 22 and the guard 33, and the electrical connectors 35 and 36 were fabricated using mechanical screws to electrically connecting the inner wires of additional coaxial cables to the second electrode 23 and the third electrode 27, respectively.

The low current and low potential output from the LCR meter were connected to the first electrical connector 34, and the high current and high potential output from the LCR meter were connected to the third electrical connector 36 via BNC cables through an electrical switch based on the Pickering Series 103, Low Capacitance SIL/SIP Reed Relays. The guard of the LCR meter was connected to the fourth electrical connector 37 and to the second electrical connector 35 via BNC cables through the electrical switch. Prior to the measurements on microorganisms, open-circuit measurements for the LCR meter were made with a 500 mL glass bottle docked into the docking station 21 and the docking screws 31 and 32 were tightened using a torque wrench with specified torque of 20 Nm; short-circuit measurements were made connecting the first electrical connector 34, the second electrical connector 35 and the third electrical connector 36 to the fourth electrical connector 37 connected to the guard of the LCR meter.

The bacteria S. *Epidermidis* were grown in BHI media in the 500 mL glass bottle at 37 °C in a temperature stabilized air bath. A Teflon coated magnet (ø8.0 mm × 30.0 mm) was immersed at the bottom of the bottle; the bottle was docked into the docking station 21 and a magnetic stirrer (IKA model RCT classic) was mounted below the docking station 21 with a stirring speed set to 200 rpm. The docking screws 31 and 32 were tightened using a torque wrench with specified torque of 20 Nm. Data of parallel impedance (change in real part of the parallel impedance, resistance ($\Delta R_p$) and change in the imaginary part of the parallel admittance, susceptance ($B_p$)) from the LCR meter were made in the frequency range from 100 Hz to 2 MHz with a sweep of eighty selected frequencies and at a voltage of 1 V.

The data of non-contact electrochemical impedance ($R_p$, $B_p$) of bacterial growth recorded as function of time were fitted by sigmoid functions ("s"-curves) according to;

$$\Delta U(t) = \frac{\Delta U_s}{1+\exp\{-(t-t_0)/\tau\}}, \quad (2)$$

where $U = \Delta R_p$ for data of parallel resistance in units of $\Omega$, and $U = \Delta B_p$ for data of parallel susceptance in units of S; $\Delta U_s$ is the data for $t=+\infty$; $\tau$ is an expontial time constant; and $t_0$ is the time at the onset of the exponential bacterial growth. The model in Eq.(2) includes a lag phase, an exponential growth phase, and a stationary phase (M. Peleg and M. G. Corradini, Critical Rev. Food Sci. Nutr., vol. 51, pp. 917-945, 2011).

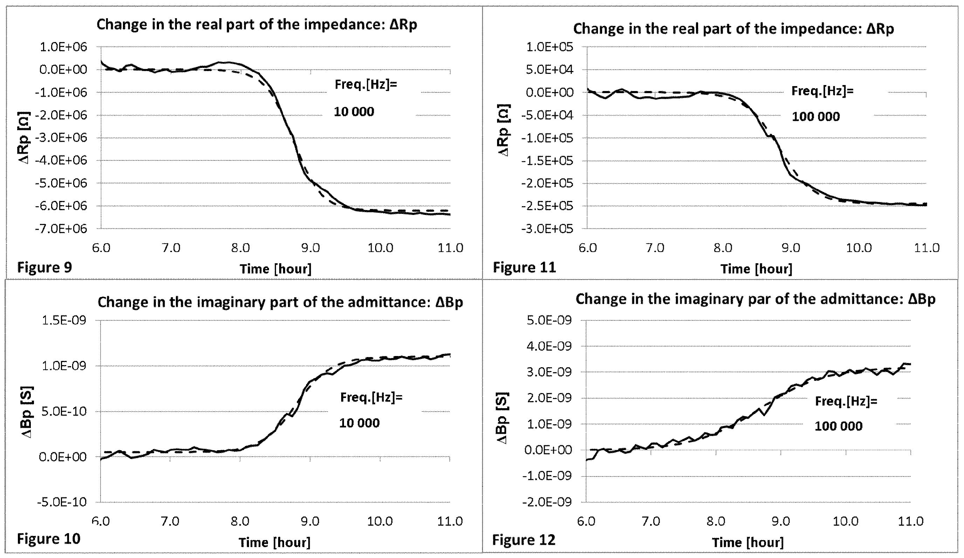

For S. Epidermidis at 37 °C, data of changes in the real part of the impedance ($\Delta R_p$) recorded between electrode 22 and electrode 27 at 10 kHz are plotted as solid curve as function of time ($t$) in Figure 9. The corresponding average temperature and standard deviation of the temperature were measured to (37.055 $\pm$ 0.026) °C. The dashed curve in Figure 9 is a fit to the data set using Eq. (2) with the parameters, $\Delta R_{p,s}$ = -6.2·10$^6$ $\Omega$, $t_0$ = 8.75 h, and $\tau$ = 0.2 h. Data of changes in the imaginary part of the admittance $\Delta B_p$ at 10 kHz are plotted as solid curve as function of time ($t$) in Figure 10. The dashed curve in Figure 10 is a fit to the data set using Eq. (2) with the parameters, $\Delta B_{p,s}$ = - 1.05·10$^{-9}$ S, $t_0$ = 8.80 h, and $\tau$ = 0.25 h. Data of changes in the real part of the impedance ($\Delta R_p$) at 100 kHz are plotted as solid curve as function of time ($t$) in Figure 11. The dashed curve in Figure 11 is a fit to the data set using Eq. (2) with the parameters, $\Delta R_{p,s}$ = -2.45·10$^5$ $\Omega$, $t_0$ = 8.83 h, and $\tau$ = 0.25 h. Data of changes in the imaginary part of the admittance $\Delta B_p$ at 100 kHz are plotted as solid curve as function of time (t) in Figure 12. The dashed curve in Figure 12 is a fit to the data set using Eq. (2) with the parameters, $\Delta B_{p,s}$ = -6.2·10$^6$ S, $t_0$ = 8.68 h, and $\tau$ = 0.50 h.

As observed in Figures 9 to 12, at both frequencies, 10 kHz and 100 kHz, there is a decrease in the real part of the impedance corresponding to a decrease in the resistance, and an increase in the imaginary part of the admittance corresponding to an increase in the capacitance as the bacteria growth. The lag phase, the exponential growth phase and the stationary phase of the bacterial growth can be observed in Figures 9-12.

Example 2

A docking system 41 being adapted to dock with non-contact electrodes adapted for real time detection of growth of microorganisms through insulated walls of a container as illustrated schematically in Figure 3 adapted for a microwell plate comprising a multitude of wells (Eppendorf® Cell Imaging Plates comprising 24 wells, each with a diameter of approximately 14 mm) was used in combination with an Agilent/Keysight 4291B impedance analyser operating from 1 MHz to 1.8 GHz combined with a computer and software as analyser. The docking system was mounted on top of the measurement station of the impedance analyser. The embodiment of the present invention of non-contact electrode configurations for real time detection of growth of microorganisms through an electrically insulated wall of a container as illustrated schematically in Figure 5 was used. The microwell plate was aligned and pressed towards a plane surface 62 by means of a lever on top of the microwell plate ensuring a sufficient force on the top side of the microwell plate 200 and the bottom side of the microwell plate 201 to reproducibly hold the microwell plate positioned with a sufficient pressure against of each set of a first electrode 110 and a second electrode 114 to a pair (111 and 115) of an RS-PRO Rounded 2-part Spring probe with a length of 25 mm, a diameter of ø1.36 mm, a head of ø1.8 mm, a spring force of 1.47 N, and a contact resistance of 30 m$\Omega$. The 2-part Spring probes were mounted in a 3D printed coaxial adaptor on top of an N-connector female, with a first electrical connector 113 and a second electrical connector 117 connected to the measurement station of the impedance analyser.

The electrodes were screen printed with conductive silver ink on top of a thin foil with an adhesive on the distal surface of the foil. The first electrode was a 4 mm disc surrounded by a second electrode comprising a ring configuration with an inner diameter of 8 mm and an outer diameter of 14 mm.

S. Epidermidis bacteria were grown in BHI media in a microwell at 37 °C in a temperature stabilized air bath. Data of series impedance (change in real part of the series impedance, resistance ($\Delta R_s$) and change in the imaginary part of the series reactance (-$\Delta X_s$, see Eq. (1)) from the impedance analyser were made in the frequency range from 1 MHz to 1 GHz with a sweep of eighty selected frequencies and at a voltage of 1 V.

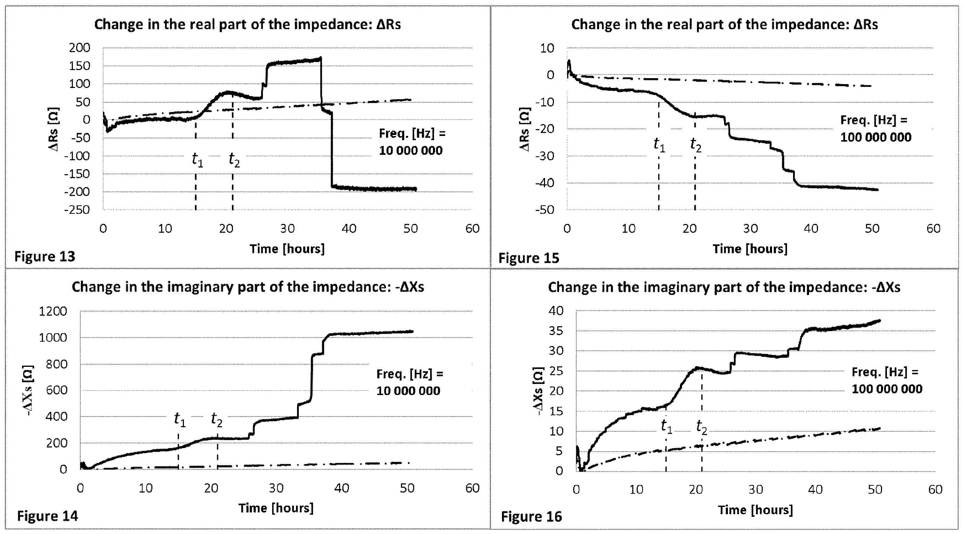

For S. Epidermidis bacteria grown in BHI media at 37 °C, data of changes in the real part of the impedance ($\Delta R_s$) recorded between electrode 110 and electrode 114 at 10 MHz are plotted as solid curve as function of time ($t$) in Figure 13. The corresponding average temperature (from t = 4 hours to t= 50 hours) and standard deviation of the temperature were measured to (37.002 $\pm$ 0.008) °C. The dot-dashed curve in Figure 13 is a plot of changes in the real part of the impedance ($\Delta R_s$) recorded between electrode 110 and electrode 114 at 10 MHz for a plain BHI media, i.e. without bacteria. The corresponding average temperature (from t = 4 hours to t= 50 hours) and standard deviation of the temperature were measured to (37.000 $\pm$ 0.004) °C. Data of changes in the imaginary part of the impedance -$\Delta X_s$ at 10 MHz are plotted as solid curve for S. Epidermidis grown in BHI media and plotted as dot-dashed curve for plain BHI media as function of time ($t$) in Figure 14. For S. Epidermidis grown in BHI media at 37 °C, data of changes in the real part of the impedance ($\Delta R_s$) recorded between electrode 110 and electrode 114 at 100 MHz are plotted as solid curve as function of time ($t$) in Figure 15. The dot-dashed curve in Figure 15 is a plot of changes in the real part of the impedance ($\Delta R_s$) recorded between electrode 110 and electrode 114 at 100 MHz for the plain BHI media. Data of changes in the imaginary part of the impedance (-$\Delta X_s$) at 100 MHz are plotted as solid curve for S. Epidermidis grown in BHI media and plotted as dot-dashed curve for plain BHI media as function of time ($t$) in Figure 16.

In Figures 13 to 16, at both frequencies, 10 MHz and 100 MHz, an exponential growth phase for S. Epidermidis (solid curves) can be observed between approximately $t_1$ = 15 hours and $t_2$ = 21 hours as indicated by the vertical dashed lines in each Figure. At both 10 MHz and 100 MHz, $-\Delta X_s$ increases during the exponential growth phase and subsequently keeps increasing. At 10 MHz, $\Delta R_s$ increases during the exponential growth phase and decreases after approximately $t$ = 35.5 hours. At 100 MHz, $\Delta R_s$ decreases during both the exponential growth phase and after this phase. The responses are observed to exhibit different behaviour at different frequencies and the non-contact impedance analysis of the present invention elucidates the complex nature of bacterial growth dynamics.

Selected embodiments of the invention:

Clause 1. A **docking** system for electrochemical detection of growth of microorganisms in a container wherein the docking system comprises

- a docking station;
- a set of electrodes comprising at least two electrodes;
- a docking arrangement comprising at least one movable element;
- a docking site for a container and
- a set of electrical connectors adapted for connecting the respective electrodes to analyser,

wherein the docking arrangement is adapted for temporarily holding a container in location relative to the set of electrodes, to provide the respective electrode to be in physical contact with an outer surface of said container at respective preselected locations, wherein the electrodes are physically separated from each other, and wherein the docking site comprises a bottom structure comprising at least one electrode of the set of electrodes.

Clause 2. The system of clause 1, wherein the docking arrangement is connected to or is connectable to the docking station.

Clause 3. The system of any one of the preceding clauses, wherein at least one of the electrodes of the set of electrodes are stationary in the docking station at a docking site to provide that the electrode comes into physical contact with the outer surface of a container when located at the docking site in the docking station, wherein the stationary electrode preferably forms part of a bottom structure of the docking site.

Clause 4. The system of any one of the preceding clauses, wherein the docking arrangement is correlated to or engaged with at least one of the electrodes of the set of electrodes, to provide the physical contact between the container outer surface and the electrode.

Clause 5. The system of clause 4, wherein the at least one movable element of the docking arrangement has a first position where at least one of the electrodes of the set of electrodes is detached from the outer surface of the container and a second position where the at least one electrode of the set of electrodes is held in physical contact with the outer surface of the container.

Clause 6. The system of clause 5, wherein the at least one movable element of the docking arrangement in the second position is adapted to establish a pressure between the at least one electrode of the set of electrodes and the outer surface of the container, preferably a pressure of at least 10 N/m$^2$, such as from 10 N/m$^2$ to 300 000 N/m$^2$.

Clause 7. The system of clause 6, wherein the at least one movable element of the docking arrangement in the second position is establishing and maintaining a pressure between the at least one electrode of the set of electrodes and the outer surface of the container for a period of at least 1 minute, such as at least 10 minutes, preferably the pressure is a preselected and/or a reproducible pressure.

Clause 8. The system of any one of the preceding clauses, wherein at least one of the electrodes of the set of electrodes is substantially plane and is preferably adapted to be in physical contact with a plane portion of the outer surface of a container, such a bottom portion of the outer surface of a container.

Clause 9. The system of any one of the preceding clauses, wherein at least one of the electrodes of the set of electrodes is circular, disc or ring shaped.

Clause 10. The system of any one of the preceding clauses, wherein at least one of the electrodes of the set of electrodes is shaped to at least partially encircling a container located at a docking site of the docking station.

Clause 11. The system of clause 10, wherein the at least one electrode of the set of electrodes comprises a strip shaped electrode adapted for encircling and contacting the outer surface of the container, wherein the strip shaped electrode is associated to the docking arrangement, such that the strip shaped electrode is detached from the outer surface of the container when the docking arrangement is in a first position and is contacting the outer surface of the container when the docking arrangement is in a second position.

Clause 12. The system of clause 11, wherein the docking arrangement comprises a ring element and an adjustment arrangement arranged for adjusting the diameter of the ring element between a first diameter at the first position and a second diameter at the second position, wherein the second diameter is larger than the first diameter.

Clause 13. The system of any one of the preceding clauses, wherein an electrical connector of the set of connectors is connected to or connectable to at least one electrode of the set of electrodes and wherein said electrical connector is at least partially located at said docking arrangement, preferably at said adjustment arrangement.

Clause 14. The system of any one of the preceding clauses, wherein said docking arrangement comprises a spring element adapted, wherein said spring element is adapted to establish a pressure between at least one of the electrodes of the set of electrodes and the outer surface of the container, such as the outer surface of a bottom area of the container.

Clause 15. The system of clause 14, wherein the docking arrangement is connected to or is connectable to the docking station, wherein the docking arrangement and the docking station is adapted for holding the container to provide that the spring element establish the pressure between the at least one electrode of the set of electrodes and the outer surface of the container, wherein the pressure preferably is at least 10 N/m$^2$, such as from 10 N/m$^2$ to 300 000 N/m$^2$.

16. The system of clause 14 or clause 15 wherein the, wherein the spring element is directly or indirectly engaged with the at least one electrode of the set of electrodes.

17. The system of clause 16, wherein the spring element is indirectly via a pin body engaged with the at least one electrode of the set of electrodes, wherein the electrode is located at the pin body or is adapted for being located at the outer surface of the container.

18. The system of any one of the preceding clauses, wherein the docking arrangement comprises at least one constraining element adapted for holding the container in temporarily fixed condition, wherein the constraining element(s) preferably is adapted for holding the container in a temporarily fixed condition while the at least one movable element of the docking arrangement to establish a pressure between at least one of the electrodes of the set of electrodes and the outer surface of the container.

19. The system of any one of the preceding clauses, wherein the system further comprises at least one electrically conductive guard adapted for shielding at least one of the electrodes of the set of electrodes.

20. The system of clause 19, wherein the electrically conductive guard has an annular shape, such as a plane ring of conductive material, wherein the electrically conductive guard preferably is connected to ground and/or to an electrical connector.

21. The system of any one of the preceding clauses, wherein the system comprises two or more sets of electrodes, each set of electrodes comprises at least two electrodes.

22. The system of any one of the preceding clauses, wherein the system is adapted for electrochemical detection of growth of microorganisms in two or more containers, such as in two or more sub-containers of a multi container device, wherein the docking arrangement is adapted for temporarily holding said containers in location relative to respective sets of electrodes, to provide the respective electrode of the sets of electrodes to be in physical contact with an outer surface of said respective containers at respective preselected locations, wherein the electrodes of respective sets of electrodes are physically separated from each other.

Clause 23. The system of any one of the preceding clauses, wherein the system further comprises at least one container, such as a single container or a multi container device, e.g. a microtiter plate.

Clause 24. The system of clause 23, wherein the container comprises container wall sections, which are of non-conductive material providing an insulated wall section, preferably the entire wall of the container is of non-conductive material.

Clause 25. The system of clause 23 or clause 24, wherein the electrodes of the set of electrodes are adapted to be in physical contact with the outer surface of said container wall at locations of the container wall which are insulated wall sections.

Clause 26. The system of any one of clauses 23-25, wherein the container is substantially free of conductive material.

Clause 27. The system of any one of clauses 23-26, wherein the system comprises a foil with a pressure sensitive adhesive adapted for being attached to the outer surface of the container, preferably at a bottom location of the container and wherein an electrode of the set of electrodes are fixed said foil.

Clause 28. The system of any one of the preceding clauses, wherein the system further comprises or is adapted to be connected to an analyser, wherein said respective electrical connectors are or are adapted for providing electrical contact with said analyser, wherein the analyser is an electrochemical analyser.

Clause 29. A method for electrochemical detection of growth of microorganisms in a sample in a container, the method comprising

- docking the container in a docking system according to any one of the preceding clauses
- providing the docking arrangement for temporarily holding the container in location relative to the set of electrodes, to provide the respective electrode to be in physical contact with an outer surface of said container at respective preselected locations, wherein the electrodes are physically separated from each other,
- connecting the respective electrical connectors to an analyser;
- performing at least one electrical measurement and
- performing the detection of growth of microorganisms based on said at least one measurement.

Clause 30. The method of clause 29, wherein the method comprises providing said electrodes of the set of electrodes to be in physical contact with the outer surface of said container wall at locations of the container wall, which are insulated wall sections.

Clause 31. The method of clause 29 or clause 30, wherein the method comprises establishing a pressure between the at least one electrode of the set of electrodes and the outer surface of the container, wherein the pressure preferably is at least 10 N/m$^2$, such as from 10 N/m$^2$ to 300 000 N/m$^2$.

Clause 32. The method of any one of clauses 29-31, wherein the step of docking the container comprises docking while the at least one movable element of the docking arrangement is in a first position and wherein the step of providing the docking arrangement for temporarily holding the container in location relative to the set of electrodes comprises providing the at least one movable element of the docking arrangement to be in a second position, wherein at least one of the electrodes of the set of electrodes is detached from the outer surface of the container when the at least one movable element of the docking arrangement is in a first position.

Clause 33. The method of any one of clauses 29-32, wherein the performing of at least one electrical measurement comprises obtaining impedance measurements data on an open-circuit-component, obtaining impedance measurements data on a short-circuit-component and obtaining impedance measurements data on a load-component and including these data in the detection of growth of microorganisms.

Clause 34. The method of any one of clauses 29-33, wherein the method further comprises determining the number and species of the microorganisms.

Clause 35. The method of any one of clauses 29-34, wherein the method further comprises performing a quantitative determination of dead microorganisms.

Clause 36. The method of any one of clauses 29-35, wherein the method comprises electrochemical detection of growth of microorganisms in two or more samples located in respective containers, such in separate containers or in a container device comprises sub-containers,

Clause 37. The method of clause 36, wherein the method comprises connecting the respective electrical connectors to the analyser via an electrical switch optionally forming part of the analyser, and wherein the method comprises directs the signals from the respective containers to be analysed in the analyser in a predefined or preselected order.

**Claims**

1. A **docking** system for electrochemical detection of growth of microorganisms in a container wherein the docking system comprises

    • a docking station;
    • a set of electrodes comprising at least two electrodes;
    • a docking arrangement comprising at least one movable element;
    • a docking site for a container
    and
    • a set of electrical connectors adapted for connecting the respective electrodes to analyser,

    wherein the docking arrangement is adapted for temporarily holding a container in location relative to the set of electrodes, to provide the respective electrode to be in physical contact with an outer surface of said container at respective preselected locations, wherein the electrodes are physically separated from each other and wherein the docking site comprises a bottom structure comprising at least one electrode of the set of electrodes, preferably the docking arrangement is connected to or is connectable to the docking station.

2. The system of claim 1, wherein at least one of the electrodes of the set of electrodes are stationary in the docking station at a docking site to provide that the electrode comes into physical contact with the outer surface of a container when located at the docking site in the docking station, wherein the stationary electrode preferably forms part of a bottom structure of the docking site, and wherein the docking arrangement is correlated to or engaged with at least one of the electrodes of the set of electrodes, to provide the physical contact between the container outer surface and the electrode, wherein the at least one movable element of the docking arrangement has a first position where at least one of the electrodes of the set of electrodes is detached from the outer surface of the container and a second position where the at least one electrode of the set of electrodes is held in physical contact with the outer surface of the container.

3. The system of claim 1 or claim 2, wherein the at least one movable element of the docking arrangement in the second position is adapted to establish a pressure between the at least one electrode of the set of electrodes and the outer surface of the container such as a pressure of at least 10 N/m$^2$, such as from 10 N/m$^2$ to 300 000 N/m$^2$, wherein the at least one movable element of the docking arrangement in the second position preferably is establishing and maintaining a pressure between the at least one electrode of the set of electrodes and the outer surface of the container for a period of at least 1 minute, such as at least 10 minutes, preferably the pressure is a preselected and/or a reproducible pressure.

4. The system of any one of the preceding claims, wherein at least one of the electrodes of the set of electrodes is substantially plane and/or wherein at least one of the electrodes of the set of electrodes is circular, disc or ring shaped and/or wherein at least one of the electrodes of the set of electrodes is shaped to at least partially encircling a container located at a docking site of the docking station, preferably the system further comprises at least one electrically conductive guard adapted for shielding at least one of the electrodes of the set of electrodes.

5. The system of any one of the preceding claims, wherein at least one electrode of the set of electrodes comprises a strip shaped electrode adapted for encircling and contacting the outer surface of the container, wherein the strip shaped electrode is associated to the docking arrangement, to provide that the strip shaped electrode is detached from the outer surface of the container when the docking arrangement is in a first position and is contacting the outer surface of the container when the docking arrangement is in a second position.

6. The system of any one of the preceding claims, wherein at least one electrode of the set of electrodes comprises a strip shaped electrode adapted for forming a ring element adapted for circling and contacting the outer surface of the container, wherein the docking arrangement comprises an adjustment arrangement arranged for adjusting the diameter of the ring element between a first diameter at a first position and a second diameter at a second position, wherein the second diameter is larger than the first diameter.

7. The system of any one of the preceding claims, wherein an electrical connector of the set of connectors is connected to or connectable to at least one electrode of the set of electrodes and wherein said electrical connector is at least partially located at said docking arrangement.

8. The system of any one of the preceding claims, wherein said docking arrangement comprises a spring element, wherein said spring element is adapted to establish a pressure between at least one of the electrodes of the set of electrodes and the outer surface of the container and , wherein the docking arrangement and the docking station is adapted for holding the container to provide that the spring element establish the pressure between the at least one electrode of the set of electrodes and the outer surface of the container, preferably the spring element is directly engaged with the at least one electrode of the set of electrodes or wherein the spring element is indirectly via a pin body engaged with the at least one electrode of the set of electrodes, wherein the electrode is located at the pin body or is adapted for being located at the outer surface of the container.

9. The system of any one of the preceding claims, wherein the docking arrangement comprises at least one constraining element adapted for holding the container in a temporarily fixed condition.

10. The system of any one of the preceding claims, wherein the system is adapted for electrochemical detection of growth of microorganisms in two or more containers, such as in two or more sub-containers of a multi container device, wherein the docking arrangement is adapted for temporarily holding said containers in location relative to respective sets of electrodes, to provide the respective electrode of the sets of electrodes to be in physical contact with the outer surface of said respective containers at respective preselected locations, wherein the electrodes of respective sets of electrodes are physically separated from each other.

11. The system of any one of the preceding claims, wherein the system further comprises at least one container, wherein the at least one container comprises one or more container wall sections, which are of non-conductive material and thereby provides insulated wall sections, preferably the entire wall of the container is of non-conductive material, preferably the electrodes of the set of electrodes are adapted to be in physical contact with the outer surface of said container wall at locations of the container wall which comprises said insulated wall sections, optionally the container is substantially free of conductive material.

12. The system of any one of the preceding claims, wherein the system comprises a foil with a pressure sensitive adhesive adapted for being attached to the outer surface of the container, preferably at a bottom location of the container and wherein an electrode of the set of electrodes are fixed to said foil.

13. The system of any one of the preceding claims, wherein the system further comprises or is adapted to be connected to an analyser, wherein said respective electrical connectors are or are adapted for providing electrical contact with said analyser, wherein the analyser is an electrochemical analyser.

14. A method for electrochemical detection of growth of microorganisms in a sample in a container, the method comprising

 • docking the container in a docking system according to any one of the preceding claims
 • providing the docking arrangement for temporarily holding the container in location relative to the set of electrodes, to provide the respective electrode to be in physical contact with an outer surface of said container at respective preselected locations, wherein the electrodes are physically separated from each other,
 • connecting the respective electrical connectors to an analyser;
 • performing at least one electrical measurement and
 • performing the detection of growth of microorganisms based on said at least one measurement,

preferably the method comprises providing said electrodes of the set of electrodes to be in physical contact with the outer surface of said container wall at locations of the container wall, which are insulated wall sections and preferably establishing a pressure between the at least one electrode of the set of electrodes and the outer surface of the container.

15. The method of claim 14, wherein the step of docking the container comprises docking while the at least one movable element of the docking arrangement is in a first position and wherein the step of providing the docking arrangement for temporarily holding the container in location relative to the set of electrodes comprises providing the at least one movable element of the docking arrangement to be in a second position, wherein at least one of the electrodes of the set of electrodes is detached from the outer surface of the container when the at least one movable element of the docking arrangement is in a first position.

16. The method of claim 14 or claim 15, wherein the performing of at least one electrical measurement comprises obtaining impedance measurements data on an open-circuit-component, obtaining impedance measurements data on a short-circuit-component and obtaining impedance measurements data on a load-component and including these data in the detection of growth of microorganisms.

17. The method of any one of claims 14-16, wherein the detection of growth of microorganisms comprises determining the number and species of the microorganisms and/or performing a quantitative determination of dead microorganisms.

18. The method of any one of claims 14-17, wherein the method comprises electrochemical detection of growth of microorganisms in two or more samples located in respective containers, wherein the method comprises connecting the respective electrical connectors to the analyser via an electrical switch optionally forming part of the analyser, and wherein the method comprises directs the signals from the respective containers to be analysed in the analyser in a selected order.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

EP 4 296 351 A1

Europäisches
Patentamt

European
Patent Office

Office européen
des brevets

# EUROPEAN SEARCH REPORT

Application Number

EP 23 17 9466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2015/042364 A1 (BACHUR JR NICHOLAS [US] ET AL) 12 February 2015 (2015-02-12) * paragraphs [0021] - [0028], [0035] - [0038]; figures 1-3,6,7 * | 1-18 | INV.<br>C12M3/00<br>C12M1/34<br>G01N27/22 |
| A | LUCHTERHAND BETTINA ET AL: "Newly designed and validated impedance spectroscopy setup in microtiter plates successfully monitors viable biomass online",<br>BIOTECHNOLOGY JOURNAL,<br>vol. 10, no. 8, 30 July 2015 (2015-07-30), pages 1259-1268, XP055776396,<br>DE<br>ISSN: 1860-6768, DOI:<br>10.1002/biot.201400534<br>* page 1261, right-hand column; figures 1A-1C * | 1-18 | |
| A,D | US 2018/237764 A1 (CAIAFA ANTONIO [US] ET AL) 23 August 2018 (2018-08-23) * paragraphs [0027] - [0029], [0035]; figure 1 * | 3,8 | |

TECHNICAL FIELDS
SEARCHED    (IPC)

C12M
G01N

The present search report has been drawn up for all claims

| Place of search | Date of completion of the search | Examiner |
|---|---|---|
| Berlin | 6 November 2023 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 9466

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2023

| Patent document cited in search report | Publication date | Patent family member(s) | | Publication date |
|---|---|---|---|---|
| US 2015042364 A1 | 12-02-2015 | EP | 1839044 A1 | 03-10-2007 |
| | | JP | 5265199 B2 | 14-08-2013 |
| | | JP | 2008525821 A | 17-07-2008 |
| | | US | 2006160171 A1 | 20-07-2006 |
| | | US | 2015042364 A1 | 12-02-2015 |
| | | WO | 2006071800 A1 | 06-07-2006 |
| US 2018237764 A1 | 23-08-2018 | US | 2018237764 A1 | 23-08-2018 |
| | | US | 2019359970 A1 | 28-11-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9531481 A
- US 8780181 B
- GB 2260407 A
- US 20060216203 A
- US 20090149334 A
- EP 3301438 A
- US 9400272 B
- US 20190359970 A

**Non-patent literature cited in the description**

- **C.E. TURICK et al.** *Appl. Microbiol. Biotechnol.,* 2019, vol. 103, 8327-8338
- **R. GNAIM et al.** *BioTechniques,* 2020, vol. 69, 27-36
- **R. NARANG et al.** *Sci. Rep.,* 2018, vol. 8, 15807
- **J. SONG et al.** *ACS Sens.,* 2020, vol. 5, 1795-1803
- **A. L. A. DE ARAUJO et al.** *Biosensors,* 2019, vol. 9 (108), 1-4
- **ZHANG et al.** *Anal. Chem.,* 2018, vol. 90 (10), 6006-6011
- **L. DELEEBEECK.** C. Thirstrup. *IEEE Trans. Instrum. Meas.,* 2021, vol. 70, 1008222
- **L. DELEEBEECK.** C. Thirstrup. *IEEE Trans Instrum Meas,* 2021, vol. 70, 1008222
- **M. PELEG ; M. G. CORRADINI.** *Critical Rev. Food Sci. Nutr.,* 2011, vol. 51, 917-945